(19) Europäisches Patentamt
European Patent Office
Office européen des brevets

(11) EP 2 926 808 A1

(12) **EUROPEAN PATENT APPLICATION**
published in accordance with Art. 153(4) EPC

(43) Date of publication:
**07.10.2015 Bulletin 2015/41**

(21) Application number: **13824127.8**

(22) Date of filing: **27.11.2013**

(51) Int Cl.:
***A61K 31/16*** *(2006.01)* ***A61K 31/203*** *(2006.01)*
***A61P 1/16*** *(2006.01)*

(86) International application number:
**PCT/ES2013/070824**

(87) International publication number:
**WO 2014/083229 (05.06.2014 Gazette 2014/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.11.2012 ES 201231850**

(71) Applicants:
- **Administración General De La Communidad Autónoma De Euskadi**
  **01010 Vitoria-Gateiz (ES)**
- **Universidad de Salamanca**
  **37008 Salamanca (ES)**

(72) Inventors:
- **BAÑALES ASURMENDI, Jesús María**
  **E-20014 San Sebastián (ES)**
- **BUJANDA PIEROLA, Luis**
  **E-20014 San Sebastián Gipuzkoa (ES)**
- **HIJONA MURUAMENDIARAZ, Elizabeth**
  **E-20014 San Sebastián Gipuzkoa (ES)**
- **MÚÑOZ GARRIDO, Patricia**
  **E-20014 San Sebastián Gipuzkoa (ES)**
- **PRIETO VALTUEÑA, Jesús**
  **E-31008 Pamplona Gipuzkoa (ES)**
- **URRIBARRI, Aura Daniela**
  **E-31008 Pamplona Gipuzkoa (ES)**
- **GARCÍA MARÍN, José Juan**
  **E-37007 Salamanca (ES)**
- **PERUGORRIA MONTIEL, María Jesús**
  **E-20014 San Sebastián Gipuzkoa (ES)**
- **LOZANO ESTEBAN, Elisa**
  **E-37007 Salamanca (ES)**

(74) Representative: **Illescas, Manuel**
**Manuel Illescas y Asociados, S.L. (MIA)**
**Principe de Vergara n° 197**
**Oficina 1°A**
**28002 Madrid (ES)**

# (54) USE OF METALLOPROTEASE INHIBITORS FOR THE TREATMENT OF POLYCYSTIC LIVER DISEASES

(57) The present invention comprises the use of metalloprotease inhibitors for the treatment of polycystic liver diseases (PLDs). The invention particularly describes the use of Marimastat as the preferred metalloprotease inhibitor for the treatment of PLDs. Treatment with Marimastat is able to inhibit liver cystogenesis by blocking metalloproteolytic hyperactivity of polycystic cholangiocytes.

EP 2 926 808 A1

## Description

## FIELD OF THE INVENTION

The present invention is in the field of medicine in general, specifically in the field of treatment of liver diseases, more specifically in the treatment of polycystic liver diseases. This invention particularly defines the use of metalloprotease inhibitors for the treatment of these pathologies.

## STATE OF THE ART

Polycystic liver diseases (PLD) are a group of genetic diseases, inherited dominantly or recessively, that can develop alone or in association with polycystic kidney diseases (PKD). Until recently, a form of isolated PLD was described, characterised by the presence of cysts only in the liver. This exclusively liver disease is called autosomal dominant polycystic liver disease (ADPLD).

ADPLD is a polycystic liver disease characterised by the presence of cysts exclusively in the liver (Masyuk T. et al. Curr Opin Gastroenterol 2009;25:265-271; Bañales JM, et al. In: Pathophysiology of the intrahepatic biliary epithelium: Research SignPost, 2008; 101-130). Twenty per cent (20%) of the patients suffering from this disease have mutations in the gene *PRKCSH* or in the gene *SEC63.* However, the type of mutation in the remaining 80% of patients with ADPLD is still unknown. The *PRKCSH* gene codes for a protein called hepatocystin. Hepatocystin is a luminal protein of the endoplasmic reticulum (ER) implicated in the control of the quality of recently synthesised glycoproteins. The *SEC63* gene codes for a membrane protein located in the endoplasmic reticulum and is involved in the machinery for protein translocation and glycosylation.

The PLDs that also affect the kidney (PKD) mainly include autosomal dominant polycystic kidney disease (ADPKD) and autosomal recessive polycystic kidney disease (ARPKD). ADPKD is the most common hereditary cystic kidney disease and affects the liver in 85% of patients. Its prevalence (between 1:600 - 1:1,000) and the number of hepatic cysts increases with age, female sex and severity of renal dysfunction (Masyuk T. et al. Curr Opin Gastroenterol 2009;25:265-271; Bañales JM, et al. In: Pathophysiology of the intrahepatic biliary epithelium: Research SignPost, 2008; 101-130). This disease is caused by mutations in the *PKD1* gene or in the *PKD2* gene. These genes code for the protein polycystin-1 (PC-1) and polycystin-2 (PC-2), which are membrane proteins that are coupled together. PC-1 is a mechanoreceptor that detects changes in biliary flow and stimulates the entry of extracellular calcium by the activation of PC-2. PC-2 is a type II glycoprotein, involved in the mobilisation of calcium between the extracellular and intracellular spaces, and between the endoplasmic reticulum and the cytoplasm.

Cystogenesis in polycystic liver diseases is a consequence of malformations in the ductal plate (Temmerman F, et al. Aliment Pharmacol Ther;34:702-713), hyper-secretory processes (Banales JsM, et al., The American Journal of Pathology 2008;173:1637) and hyper-proliferative processes (Banales JM, et al. Hepatology 2009;49:160-174) and changes in mRNA patterns (Lee S-O, Masyuk T, et al. The Journal of Clinical Investigation 2008;118:3714) of cholangiocytes. In addition, these changed processes are intra-cellularly associated with an increase of cAMP and a decrease of $Ca^{2+}$ (Banales JM, et al. Hepatology 2009;49:160-174; Masyuk TV, et al. Gastroenterology 2007;132:1104-1116). In this sense, an increase in the cAMP levels in both cholangiocytes and serum of PCK rats in comparison with normal rats has been demonstrated (Masyuk TV, et al. Molecular Biology of the Cell. 2007). This event is associated with an increase in liver cystogenesis, which is inhibited after the administration of octreotide, an analogue of somatostatin, which reduces the intracellular levels of cAMP by activating the intracellular phosphodiesterases.

The most common symptoms and complications that patients with PLDs develop are hypertension, back pain, distension and abdominal discomfort, dyspnea, gastroesophageal reflux, bleeding, infection and/or rupture of the cysts (Masyuk TV. et al. Curr Opin Gastroenterol 2009;25:265-271; Bañales JM, et al. In: Pathophysiology of the intrahepatic biliary epithelium: Research SignPost, 2008; 101-130). Until relatively recently, there was no medical treatment for PLDs and the only definitive treatment in patients with large cysts in the liver continues to be liver transplant. Other surgical therapies include a wide variety of procedures for reducing the volume of the liver such as, for example, aspiration and scleratotherapy, fenestration and segmental liver resection. However, in addition to being invasive, these therapies are only partially effective, are associated with considerable morbidity and mortality and in the end, the cysts appear again. In recent years, new non-invasive techniques have been developed for the treatment of the disease directed at the inhibition of growth of cholangiocytes, as well as inhibition of their secretory activity (Temmerman F, et al. Aliment Pharmacol Ther. 2011 Oct;34(7):702-13). Among these techniques, treatment with somatostatin analogues such as, for example octreotide and lanreotide, are particularly noteworthy. These inhibitors are able to inhibit the increase in cAMP levels, also reducing secretion of cystic fluid and the proliferation of the cholangiocytes, resulting in the reduction of liver cysts, as demonstrated in clinical trials in which these analogues were tested. However, the percentage inhibition of liver cysts is very low, only around ~5% (van Keimpema L, et al. Gastroenterology 2009; 137:1661-8; Hogan M, et al. J Am Soc Nephrol 2010; 21: 1052-61), so the search for more effective therapies is necessary.

Another therapeutic strategy used in the treatment of PLDs is the use of mTOR (*mammalian target of rapamycin*) inhibitors such as sirolimus, tacrolimus y everolimus. This class of drugs has strong antiproliferative effects and has become an important part of immunosuppressive therapy after organ transplant. Clinical trials performed with these mTOR inhibitors have shown contradictory results. Whereas treatment with sirolimus in a group of ADPKD patients that were subjected to kidney transplant and had polycystic livers reduced the volume of the liver, by contrast, treatment with tacrolimus produced the opposite effect, that is it increased the volume of the liver (Qian Q, et al. J Am Soc Nephrol 2008;19: 631-8; Serra AL, et al. N Engl J Med 2010; 363: 820-9; Walz G, et al. N Engl J Med 2010; 363: 830-40). Therefore there is no consensus on the use of mTOR inhibitors for the treatment of PLDs.

Thus, apart from liver transplant, none of the options currently available have modified the natural course of the PLDs. Furthermore, there is no consensus over the optimum time and procedure in which this should be performed. Thus, prevention and an improvement in clinical symptoms of these patients would provide them with undeniable benefit.

## DESCRIPTION OF THE INVENTION

### Brief description of the invention

To overcome the problems in the state of the art in relation to the provision of an effective treatment for PLDs, the present invention describes the use of metalloprotease inhibitors for the treatment of these pathologies.

Matrix metalloproteases (MMP) are a family of endopeptidases depending on zinc in their active site that participate in the degradation and remodelling of the extracellular matrix (ECM). These proteins are key, not only in biological processes such as embryonic development and homoeostasis of the tissues, but also in pathological processes such as cancer and fibrogenic processes. The MMPs are traditionally classified according to their structure and/or the specificity of the ECM substrate that they degrade. According to these parameters, the MMPs are organised into six groups: collagenases, gelatinases, stromelysins, matrilysins, metalloproteases associated with the membrane and "other MMPs". The activity of the various MMPs is regulated by a series of natural endogenous inhibitors, the tissue inhibitors of metalloproteinases (TIMP), but there are also various synthetic MMP inhibitors such as, for example, pseudopeptide-type inhibitors also known as peptide mimetics, or non-peptide inhibitors derived from tetracycline and bisphosphonates. All of these bind directly to the zinc binding site of these molecules and therefore interact directly with the catalytic site of the MMPs, blocking their proteolytic activity. In this sense, the international application WO 2004/110974 describes compounds and their physiologically functional derivatives and their use as metalloproteinase inhibitors. In this sense, documents WO 2004/113279, WO 2005/026120, US 6.350.885 and WO 98/09940 also reveal different types of MMP inhibitors and their uses for the treatment of general inflammatory pathologies. There are other documents in the state of the art describing various MMP inhibitors, all of them characterised by the most important structural requirement that a MMP inhibitor must have, which is the presence in the structure of a zinc binding group (ZBG) able to chelate ionic zinc at the active site (see Figure 29). For purposes of the present invention, the zinc binding group in the MMP inhibitors is able to chelate the active site of the binding of MMPs to the zinc ion, thereby inhibiting their proteolytic activity. The main ZBGs used include: hydroxamates, formylhydroxylamines, sulfhydryls, phosphinates, amino carboxylates, and carboxylates, among others.

For purposes of the present invention, the term MMP inhibitor refers to any molecule, natural or synthetic or any of its derivatives or analogues, both structural and functional, which incorporate a zinc binding group in the structure that is able to chelate the active binding site of MMPs to the zinc ion, thereby inhibiting the proteolytic activity of MMPs. For purposes of the present invention, the following documents are included by reference: Whittaker M., et al. Celltransmissions Vol 17 (1): 3-12; Fisher JF et al. Cancer Metastasis Rev. 2006; 25:115-136; that describe various MMP inhibitors, all characterised by comprising a zinc binding group in their molecules and inhibiting the catalytic activity of MMPs.

For purposes of the present invention, pseudopeptide inhibitors, also known as peptide-mimetic inhibitors of MMPs, have a structure that mimics the substrate lysis site of MMPs. These compounds bind reversibly at the MMP active site in a stereospecific way and chelate the zinc atom at the enzyme activation site. Various groups able to bind the zinc atom have been tested as MMP inhibitors by binding to the active site of these enzymes; these groups include carboxylates, aminocarboxylates, sulfhydryls, derivatives of phosphoric acid and hydroxamates. The majority of MMP inhibitors in clinical development are hydroxamate derivatives. We can include retinoic acid or vitamin A, together with its analogues and/or derivatives, within the group of pseudopeptide inhibitors or peptide mimetics described in the present invention. The pseudopeptide inhibitors of MMPs are compounds derived from hydroxamates, the most well-known being Batimastat (BB-94) and Marimastat (BB-2516). The pseudopeptide inhibitor preferably used in the present invention is Marimastat (BB-2516). Marimastat is a pseudopeptide inhibitor of MMPs with a wide spectrum and low molecular weight. It has better oral bioavailability and less toxicity that Batimastat (Rothenberg ML. et al. Oncologist. 1998;3(4):271-274; Singhmura S. The Clinical Research Plus. May 2011. (2):1). It is absorbed rapidly by the intestine and is well tolerated, with an elimination half-life of 8-10 hours. Marimastat is a potent and reversible MMP inhibitor and its action affects interstitial collagenase (MMP-1), gelatinase A (MMP-2), gelatinase B (MMP-9), matrilysin (MMP-7),

stromelysin-1 (MMP-3) and metalloelastase (MMP12).

For purposes of the present invention, the MMP non-peptide inhibitors have a similar mode of action to the pseudopeptide inhibitors but have greater specificity for the type of metalloprotease. Their spectrum of action is more selective, with weak activity against MMP-1, but strong activity against metalloprotease-2 and 9. Included in the main molecules of this category are Prinomastat (AG3340), which is a selective inhibitor for metalloproteases 2, 3, 9, 13 and 14; BAY-12-9566, which has a long half-life in plasma of between 90-100 hours and an extremely high binding fraction to plasma proteins (>99.99%) (Rothenberg ML. et al. Oncologist. 1998;3(4):271-274). The latter has not until now been used in the clinic, but is presented as an effective alternative as an adjuvant to surgery in the treatment of breast cancer. Other compounds belonging to this group are: BMS-275291 and MMI270 (CGS27023A).

Within the group of MMP inhibitors that are derivatives of tetracycline are compounds that not only inhibit the activity of the MMPs but also the production of these enzymes. Derivatives of tetracycline inhibit collagenases, MMP-1, -3 and -13, and the gelatinases, MMP-2 and -9, through multiple mechanisms, including 1) blocking the activity of mature MMPs by chelation of the zinc atom in the enzyme binding site, 2) interfering with proteolytic activation of pro-MMPs in their active form, 3) reducing the expression of MMPs and 4) protecting the MMPs from proteolytic and oxidative degradation. Derivatives of tetracycline include classical antibiotics such as tetracycline, doxycycline, minocycline and also new analogous compounds such as Metastat (Col-3).

The bisphosphonates are molecules able to inhibit the MMPs initially used in disorders of calcium balance, and later in the palliative treatment of osseous metastases. These molecules act by reducing proteolytic activity and reducing secretion of MMPs. Clodronate is one the most frequently used bisphosphonates.

As used in this document, the term "analogue or derivative" refers to any molecule that is known to act in a similar way to that to which reference is made with the term "analogue or derivative". For example, the phrase "a derivative of retinoic acid or an analogue of retinoic acid" refers to any structural derivative of retinoic acid or to a functional analogue of this acid.

Thus the invention comprises the use of metalloprotease inhibitors that comprise in their structure a zinc binding group for the preparation of a pharmaceutical composition for the treatment of PLDs. Specifically, the present invention describes the use of a MMP inhibitor of the pseudopeptide type, preferably Marimastat, for the preparation of a pharmaceutical composition for the treatment of PLDs, able to specifically inhibit hepatic cystogenesis by blocking metalloproteolytic hyperactivity of the polycystic cholangiocytes, indicating a reduction in the progression of the PLDs, without showing any therapeutic or beneficial effect on renal cystogenesis.

MMP inhibitors, as demonstrated in the present invention, have the ability to cause a decrease in levels of fibrosis and deposition of peribiliary collagen in subjects treated with these inhibitors compared to control untreated subjects.

For purposes of the present invention, the term PLD refers to a group of pathologies or genetic disorders that progress with the presence of cysts exclusively in the liver of patients suffering from these types of pathologies.

Another object of the present invention refers to a pharmaceutical composition that comprises at least one metalloprotease inhibitor that comprises a zinc binding group in its structure, together with pharmaceutically acceptable vehicles or excipients. In a preferred embodiment, the composition of the invention can also comprise another active principle. In a more preferred embodiment, these active ingredients are selected from: somatostatin and its analogues, preferably octreotide (CAS No. 83150-76-9), lanreotide (CAS No. 108736-35-2), vapreotide (CAS No. 103222-11-3) and/or pasireotide (CAS No. 396091-73-9) and/or ursodeoxycholic acid (CAS No. 128-13-2).

Another object described in the present invention refers to a method for the treatment of PLDs characterised by the administration to a subject suffering from this disease of a therapeutically effective amount of at least one metalloprotease inhibitor, as described in the present invention, or of a pharmaceutical composition, as previously described.

The content of all the references cited, including the bibliographic references, the patents cited throughout the present document, and the references that are listed below are expressly included by reference in the present application. In case of conflict, the definitions included in the present document will prevail.

**Description of the figures**

**Figure 1.** Analysis of the expression at the level of mRNA of specific markers of cholangiocytes in *in vitro* cultures of normal human cholangiocytes (control) and polycystic cholangiocytes (ADPKD). CK7: cytokeratin 7, CK19: cytokeratin 19, AQP1: aquaporin 1 and AE2: ($Cl^-/HCO3^-$ exchanger).

**Figure 2.** Basal metalloproteolytic activity of normal human cholangiocytes (control) (black bars) and polycystic cholangiocytes (white bars) in culture. GM6001: MMP inhibitor, which is used as a specificity control of the assay; recombinant MMP-10: positive control; and DMEM/F12 culture medium without serum: negative control. n = 6 in

each study group [control groups (i.e. GM6001, recombinant MMP-10, and culture medium alone: n = 1)].

**Figure 3.** Analysis of the expression at the level of mRNA of MMPs with collagenase activity (MMP-1, -8, -13 and -18) in normal human cholangiocytes (control) (black bars) and polycystic cholangiocytes (white bars) in culture. n = 6 in each study group; n.s: not significant.

**Figure 4.** Analysis of expression at the level of mRNA of MMPs with gelatinase activity (MMP-2 and -9) in normal human cholangiocytes (control) (black bars) and polycystic cholangiocytes (white bars) in culture. n = 6 in each study group; n.s: not significant.

**Figure 5.** Analysis of expression at the level of mRNA of MMPs with stromelysin activity (MMP-3, -10, -11 and -27) in normal human cholangiocytes (control) (black bars) and polycystic cholangiocytes (white bars) in culture. n = 6 in each study group; n.s: not significant.

**Figure 6.** Analysis of expression at the level of mRNA of MMPs with matrilysin activity (MMP-7) in normal human cholangiocytes (control) (black bars) and polycystic cholangiocytes (white bars) in culture. n = 6 in each study group.

**Figure 7.** Analysis of the expression at the level of mRNA of membrane-type MMPs (MMP-14, -15, -16, -17, -24 and -25) in normal human cholangiocytes (control) (black bars) and polycystic cholangiocytes (white bars) in culture. n = 6 in each study group.

**Figure 8.** Analysis of expression at the level of mRNA of natural inhibitors of MMPs, TIMPs (TIMP-1, -2, -3 and -4), in normal human cholangiocytes (control) (black bars) and polycystic cholangiocytes (white bars) in culture. n = 6 in each study group.

**Figure 9.** Metalloproteolytic activity of normal human cholangiocytes (control) (black bars) and polycystic cholangiocytes (white bars) in culture, in the presence of hormones, cytokines and growth factors. (n = 5 in each study group). IL-8: interleukin 8; IL-6 interleukin 6, 17β-Es: 17β-estradiol; VEGF: endothelial vascular growth factor; HGF: hepatocyte growth factor; EGF epidermal growth factor; GROα: growth regulator of oncogene alpha and ENA-78: epithelial neutrophil-activating peptide-78.

**Figure 10.** Analysis of expression at the level of mRNA of MMP-2 in normal human cholangiocytes (control) (black bars) and polycystic cholangiocytes (white bars) in culture in the presence of IL-6, IL-8 or 17β-estradiol. (n = 6 in each study group).

**Figure 11.** Analysis of expression at the level of mRNA of MMP-3 in normal human cholangiocytes (control) (black bars) and polycystic cholangiocytes (white bars) in culture in the presence of IL-6, IL-8 or 17β-estradiol. (n = 6 in each study group).

**Figure 12.** Analysis of expression at the level of mRNA of MMP-7 in normal human cholangiocytes (control) (black bars) and polycystic cholangiocytes (white bars) in culture in the presence of IL-6, IL-8 or 17β-estradiol. (n = 6 in each study group).

**Figure 13.** Analysis of expression at the level of mRNA of MMP-10 in normal human cholangiocytes (control) (black bars) and polycystic cholangiocytes (white bars) in culture in the presence of IL-6, IL-8 or 17β-estradiol. (n = 6 in each study group).

**Figure 14.** Analysis of expression at the level of mRNA of MMP-11 in normal human cholangiocytes (control) (black bars) and polycystic cholangiocytes (white bars) in culture in the presence of IL-6, IL-8 or 17β-estradiol. (n = 6 in each study group).

Figure 15. Analysis of expression at the level of mRNA of MMP-13 in normal human cholangiocytes (control) (black bars) and polycystic cholangiocytes (white bars) in culture in the presence of IL-6, IL-8 or 17β-estradiol. (n = 6 in each study group).

**Figure 16.** Analysis of expression at the level of mRNA of MMP-15 in normal human cholangiocytes (control) (black bars) and polycystic cholangiocytes (white bars) in culture in the presence of IL-6, IL-8 or 17β-estradiol. (n = 6 in each study group).

**Figure 17.** Analysis of expression at the level of mRNA of MMP-17 in normal human cholangiocytes or control (black bars) and polycystic cholangiocytes (white bars) in culture in the presence of IL-6, IL-8 or 17β-estradiol. (n = 6 in each study group).

**Figure 18.** Analysis of expression at the level of mRNA of MMP-27 in normal human cholangiocytes (control) (black bars) and polycystic cholangiocytes (white bars) in culture in the presence of IL-6, IL-8 or 17β-estradiol. (n = 6 in each study group).

**Figure 19.** Metalloproteolytic activity of polycystic human cholangiocytes in culture in the presence of anti-IL-6R antibodies (bar with oblique lines) and anti-CXCR1 antibodies (white bar) or both (cross-hatched bar). A secondary goat antibody that recognises IgGs of rabbit (black bar) was used as a negative control. (n = 12 in each study group).

**Figure 20.** Metalloproteolytic activity of normal human cholangiocytes in culture in the presence of anti-IL-6R antibodies (bar with oblique lines) and anti-CXCR1 antibodies (white bar) or both (cross-hatched bar). A secondary goat antibody that recognises IgGs of rabbit (black bar) was used as a negative control. (n = 6 in each study group).

**Figure 21.** Basal metalloproteolytic activity of normal human cholangiocytes and polycystic human cholangiocytes in culture in the presence of 13-cis-retinoic acid **(A)** and Marimastat **(B).** Black bars: normal human cholangiocytes (control); white bars: polycystic human cholangiocytes; bars with black cross-hatching: normal human cholangiocytes (control) treated with 13-cis-retinoic acid **(A)** or Marimastat **(B);** bars with white bricks: polycystic human cholangiocytes treated with 13-cis-retinoic acid **(A)** or Marimastat **(B)** (n = 6 in each study group).

**Figure 22.** Stained photographs obtained by immunohistochemical techniques showing the expression of MMP-1, -2 and -3 in hepatic tissue of normal individuals and of patients with PLDs (ADPKD and ARPKD) (optical microscopy: magnification 20X).

**Figure 23.** Photographs of stains obtained by immunohistochemical techniques showing the expression of MMP-1, -2 and -3 in liver tissue of normal rats and of PCK polycystic rats. (Optical microscopy: magnification 20X).

**Figure 24. A)** Representative images of control rat liver and rat liver treated with Marimastat. **B)** Quantitative analysis of the presence of hepatic cystogenesis in PCK rats using the Image-J program (*U.S. National Institute of Health*). (n = 11 animals in the control group (black circles) and 9 in the group treated with Marimastat (white circles)).

**Figure 25. A)** Representative images of control rat kidney and kidney of rat treated with Marimastat. **B)** Quantitative analysis of the presence of renal cystogenesis in PCK rats using the Image-J program (*U.S. National Institute of Health*) (n = 11 animals in the control group (black circles) and 9 in the group treated with Marimastat (white circles)).

**Figure 26. A)** Representative images of Picro-Sirius Red Stain in liver of control rat and liver of PCK rat treated with Marimastat. **B)** Representative images of Picro-Sirius Red Stain in kidney of control rat and kidney of PCK rat treated with Marimastat.

**Figure 27. A)** Analysis of the expression at protein level (Western blot) of the cholangiocytes marker Ck19 in hepatic samples of control rats and PCK rats treated with Marimastat. **B)** Analysis of the expression at the level of mRNA of the collagen 1a1 marker in hepatic samples of normal rats compared to PCK rats treated or not treated with Marimastat.

**Figure 28.** Analysis of the expression at the level of mRNA of the markers IL-6 **(A)** and CXCL 1 **(B)** in liver samples of normal rats compared to PCK rats treated or not treated with Marimastat.

**Figure 29.** Design of matrix metalloproteinase inhibitors based on the sequence of the site of excision of the collagen substrate.

**Detailed description of the invention**

One of the objects described in the present invention refers to the use of at least one MMP inhibitor comprising a zinc binding group in its structure for the manufacture of a composition for the treatment of PLDs, or alternatively, it refers to MMPs inhibitors, preferably at least one MMP inhibitor, comprising a zinc binding group in its structure for use in the treatment of PLDs.

In a preferred embodiment, the metalloprotease inhibitors can be selected from pseudopeptide, non-peptide, tetracycline derivative and/or bisphosphonate inhibitors, preferably pseudopeptide inhibitors.

In another preferred embodiment, the pseudopeptide MMP inhibitors are selected from any in the list: Marimastat and/or retinoic acid and/or any of their analogues and/or derivatives, preferably Marimastat.

In another preferred embodiment, the non-peptide MMP inhibitors are selected from any in the list: Prinomastat, BMS-275291 and/or MMI270.

A list of various metalloprotease inhibitors that can be used in the present invention are shown below. (IUPAC: International Union of Pure and Applied Chemistry).

| Name | IUPAC name | Formula |
|---|---|---|
| Marimastat | N-[2,2-dimethyl-1-(methylcarbamoyl)propyl]-2-[hydroxy-(hydroxycarbamoyl)methyl]- 4-methyl-pentanamide | 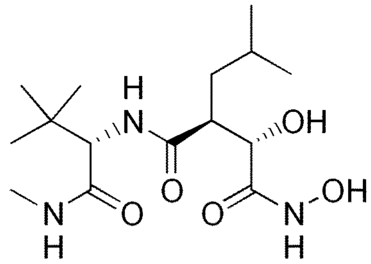 |
| Batimastat | (2R,3S)-N4-Hydroxy-2-isobutyl-N1-[(2S)-1-(methylamino)-1-oxo-3-phenyl-2-propanyl]-3-[(2-thienylsulfanyl)methyl]succinamide | 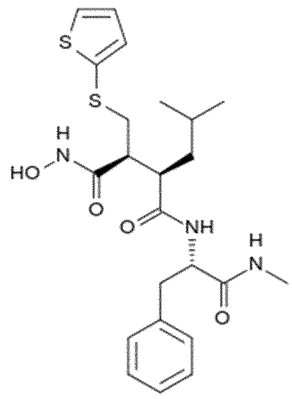 |
| Retinoic acid | (2E,4E,6E,8E)-3,7-dimethyl-9-(2,6,6-trimethylcyclohexen-1-yl)nona- 2,4,6,8-tetraenoic acid | 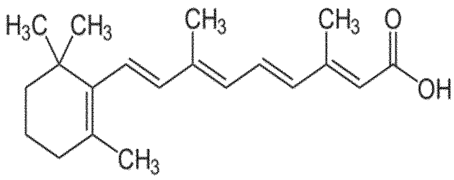 |
| Prinomastat | (S)-N-hydroxy-2,2-dimethyl-4-((4-(pyridin-4-yloxy)phenyl) sulfonyl)thiomorpholine-3-carboxamide | 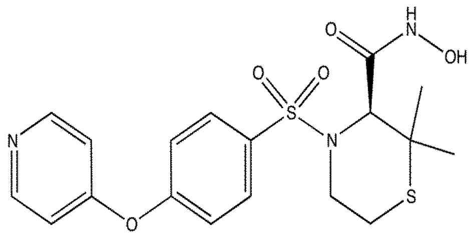 |
| Rebimastat (BMS-275291) | (S)-N-((S)-1-amino-3,3-dimethyl-1-oxobutan-2-yl)- 2-((S)-2-mercapto-4-(3,4,4-trimethyl-2,5-dioxoimidazolidin-1-yl)butanamido)- N,4-dimethylpentanamide | 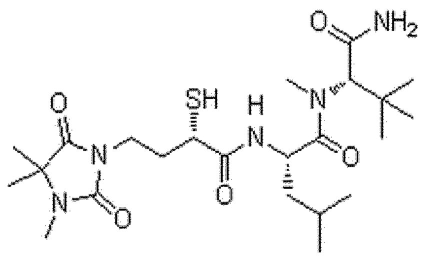 |

(continued)

| Name | IUPAC name | Formula |
|---|---|---|
| Tetracycline | (2Z,4S,4aS,5aS,12aS)-2-(amino-hydroxymethylidene)-4-dimethylamino-6,10,11,12a-tetrahydroxy-6-methyl-4,4a,5,5a-tetrahydrotetracene-1,3,12-trione | 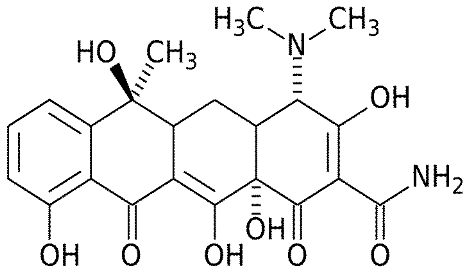 |

Another of the objects described in the present invention refers to a pharmaceutical composition comprising at least one metalloprotease inhibitor, as defined throughout the present invention, together with pharmaceutically acceptable vehicles or excipients.

In a preferred embodiment, the composition of the invention can also comprise another active principle. In a more preferred embodiment, these active ingredients are selected from any of the following: somatostatin and its analogues and/or ursodeoxycholic acid (CAS No. 128-13-2). The analogues of somatostatin are preferably selected from any of the following: octreotide (CAS No. 83150-76-9), lanreotide (CAS No. 108736-35-2), vapreotide (CAS No. 103222-11-3) and/or pasireotide (CAS No. 396091-73-9).

In another preferred embodiment, the pharmaceutical composition of the invention is characterised in that the metalloprotease inhibitor is selected from: pseudopeptide inhibitors, non-peptide inhibitors, tetracycline derivatives and/or bisphophonates. The pseudopeptide inhibitors are preferably selected from any of the list: Marimastat and/or retinoic acid or any of their analogues. In a more preferred embodiment of the composition of the invention, the metalloprotease inhibitor is Marimastat.

In another preferred embodiment, the non-peptide MMP inhibitors are selected from any in the list: Prinomastat, BMS-275291 and/or MMI270.

In another preferred embodiment of the composition of the invention, the active principle, which can accompany the MMP inhibitor or inhibitors, is selected from the following: somatostatin and its analogues, preferably octreotide (CAS No. 83150-76-9), lanreotide (CAS No. 108736-35-2), vapreotide (CAS No. 103222-11-3) and/or pasireotide (CAS No. 396091-73-9) and/or ursodeoxycholic acid (CAS No. 128-13-2).

As used here, the term "active principle", "active substance", "pharmaceutically active substance", "active ingredient" or "pharmaceutically active ingredient" refers to any component that potentially provides pharmacological activity or other effect in the diagnosis, cure, mitigation, treatment or prevention of a disease, or that affects the structure or function of the body of a human or other animals. The term includes components that promote a chemical change in the preparation of the drug and that are present in it in a modified form, thereby providing a specific activity or effect.

Another of the objects described in the present invention refers to the use of the pharmaceutical composition described in the present invention for the treatment of PLDs, or alternatively, the pharmaceutical composition described in the present invention for use in the treatment of PLDs.

Pharmaceutical compositions of the present invention can be formulated for administration to an animal, and more preferably to a mammal, including a human, in a variety of forms known in the state of the art. Thus they can be, without limitation, in sterile aqueous solution or in biological fluids such as serum. The aqueous solutions can be buffered or not buffered and may have additional active or inactive components. The additional components include salts to modulate ionic force, preservatives including, but without limitation, antimicrobial agents, antioxidants, chelating agents and similar, and nutrients including glucose, dextrose, vitamins and minerals. Alternatively, the compositions can be prepared for administration in solid form. The compositions can be combined with various vehicles or inert excipients including, without limitation; agglutinants such as microcrystalline cellulose, Tragacanth or gelatin; excipients such as starch or lactose; dispersing agents such as alginic acid or maize starch; lubricants such as magnesium stearate, glidants such as colloidal silicon dioxide; sweetening agents such as sucrose or saccharine; or flavouring agents such as mint or methyl salicylate.

Such compositions and/or their formulations can be administered to an animal, including a mammal, and therefore a human, in a variety of ways, including, without limitation, intraperitoneally, intravenously, intramuscularly, subcutaneously, intrathecally, intraventricularly, orally, enterally, parenterally, intranasally and dermally. The route of administration is preferably oral.

The dose required to obtain a therapeutically effective amount depends on a variety of factors such as, for example, age, weight, sex, tolerance, etc. of the mammal. In the meaning used in this description, the expression

"therapeutically effective amount" refers to the amount of compounds, in the case of the present invention it refers to the amount of metalloprotease inhibitor or of the active principle that can accompany it, or salts thereof, prodrugs, derivatives or analogues, or combinations thereof, that cause the desired effect and, in general, will be determined by characteristics of these prodrugs, derivatives or analogues and the desired therapeutic effect. The "adjuvants", "excipients" and "pharmaceutically acceptable vehicles" that can be used in these compositions are vehicles known by experts in the field.

The terms "adjuvant", "excipient", "additive" or any of their synonyms refer to a substance that aids the absorption, distribution or action of any of the active principles of the present invention, stabilises this active substance or aids in the preparation of the medicine in the sense of giving it consistency or providing flavours to make it more palatable. Thus, the excipients can have the function of maintaining the ingredients bound such as, for example, starches, sugars or celluloses, the function of sweetening, the function of colouring, the function of protecting the medicine such as, for example, isolating it from air and/or humidity, the function of filling a tablet, capsule or any other form of presentation such as, for example, dibasic calcium phosphate, a disintegration function to facilitate solution of the components and their absorption in the intestine, without excluding other types of excipients not mentioned in this paragraph.

The term "pharmaceutically acceptable excipient" refers to an excipient that is permitted and evaluated so that it does not cause harm to the organisms to which it might be administered.

In addition, the excipient must be pharmaceutically suitable, that is, an excipient that allows the activity of the active principle or the active principles, that is, compatible with the active principle, in this case, the active principle is any of the compounds of the present invention.

A "pharmaceutically acceptable vehicle" refers to a substance or combination of substances, known in the pharmaceutical sector, used in the preparation of pharmaceutical forms of administration and includes, without limitation, solids, liquids, solvents or surfactants.

The vehicle, just as the excipient, is a substance used in medicines to dilute any of the compounds of the present invention to a specific volume or weight. A pharmaceutically acceptable vehicle is an inert substance or a substance with an action that is analogous to any of the items of the present invention. The function of the vehicle is to facilitate the incorporation of other compounds, to enable better dosing and administration or to give consistency and form to the pharmaceutical composition. When the form of presentation is liquid, the pharmaceutically acceptable vehicle is the diluent.

Another of the objects described in the present invention refers to a method of treatment of PLDs that comprises the administration to a subject suffering from this disease of a therapeutically effective amount of at least one MMP inhibitor or of a pharmaceutical composition, as described in the present invention. A "therapeutically effective amount" is considered to mean the minimum amount of these inhibitors necessary to reverse the disease.

In a preferred embodiment, the method of treatment described in the present invention is characterised in that the MMP inhibitors can be selected from pseudopeptide, non-peptide, tetracycline derivatives and/or bisphosphonates, with the preferred inhibitors being pseudopeptides.

In another preferred embodiment, the treatment method described in the present invention is characterised in that the pseudopeptide inhibitors are selected from any of the list: Marimastat and/or retinoic acid and/or any of its analogues and/or derivatives, preferably Marimastat.

In another preferred embodiment, the treatment method described in the present invention is characterised in that the non-peptide MMP inhibitors are selected from any of the list: Prinomastat, BMS-275291 and/or MMI270.

The MMP inhibitors described in the present invention can be used as active principles of drugs for the treatment in human patients or in animals and can be prepared in formulations and/or administered in accordance with existing knowledge in the state of the art of pharmaceutical development in various ways such as: orally, intramuscularly, intraperitoneally, intradermally or by capsules, lozenges or tablets, in the form of the salts.

The term "individual" or "subject" as used in the description refers to animals, preferably mammals, and more preferably humans. The term "individual" or "subject" is not limiting in any aspect, so that this may be of any age, sex or physical condition.

Throughout the description and the claims, the word "comprise" and its variants must be interpreted in an inclusive sense, in contrast to an exclusive or exhaustive sense, such as for example, the term "includes" or "consisting in". That is, the term "comprise" must be interpreted in the sense of "include but not limited to", whereas the term "include" or "consisting in" must be interpreted in the sense of "include and limited to". Therefore the word "comprise" and its variants does not exclude other technical characteristics, components or steps.

For experts in the field, other objects, advantages and characteristics of the invention will emerge, partly from the description and partly from the practice of the invention. The following examples and figures are provided for illustration purposes only and are not to be considered as limiting the present invention.

## EXAMPLES

***Example 1.*** ***Isolated and cultured cells of normal and polycystic livers show markers of differentiated cholangiocytes.***

*Isolation and culture of human cholangiocytes*

Normal human cholangiocytes were isolated from a sample of normal liver tissue of a woman who suffered surgical excision of a local liver adenoma in the Mayo Clinic (Rochester, MN, USA). Isolation of polycystic cholangiocytes was performed from a liver explant of a woman who suffered a hepatorenal transplant due to advanced ADPKD in the University Clinic of Navarra, Spain. The protocol for isolation of the cholangiocytes was that described in Banales JM, et al. Hepatology;56:687-697. The isolated bile ducts were seeded in culture flasks covered with a fine layer of type I collagen (Cellstar, Greiner Bio-One, Frickenhausen, Germany) and DMEM/F-12 enriched medium (non-essential amino acids, lipid concentrate, vitamins, L-glutamine, trypsin inhibitor, foetal bovine serum, dexamethasone, epidermal growth factor, bovine pituitary extract, triiodothyronine, insulin and forskolin). These cultures were maintained in enriched DMEM/F-12 medium in an incubator at 37 °C and 5% $CO_2$, with the medium being refreshed every 48 hours.

Analysis of the cellular phenotype by optical phase contrast microscopy showed that both types of isolated cholangiocytes, from normal liver and polycystic liver, showed polygonal morphology characteristic of this cell type.

*Analysis of expression levels of various cholangiocyte markers at the level of mRNA*

Purity of the normal human cholangiocyte and human polycystic cholangiocyte cultures was checked by analysing the expression by mRNA quantification of various genes characteristic of cholangiocytes such as cytokeratin 19 (CK19), cytokeratin 7 (CK7), aquaporin 1 (AQP1) and the anion exchanger ($Cl^-HCO_3^-$) AE2. To perform the analysis of gene expression levels, the total RNA of normal and polycystic human cholangiocytes in culture was isolated. Next, reverse transcription of the mRNA to cDNA was performed followed by polymerase chain reaction (PCR). Total RNA from cholangiocytes was isolated using TriReagent (Sigma-Aldrich) according to the manufacturer's protocol. Quantification of the RNA was performed by UV spectrophotometry using NanoDrop® ND-1000 (Thermo Scientific, DE, USA) equipment.

From 1 μg of total extracted RNA, cDNA was obtained by inverse or reverse transcription (i.e. retrotranscription, RT). The RT reaction was performed in an Applied Biosystems 2720 Thermal Cycler following the manufacturer's instructions. The cDNA obtained was then finally diluted to a final volume of 200 μl in Braun sterile water for injection (Braun Medical, Barcelona, Spain). Finally, 4 μl of this cDNA dilution was used as a template for the PCR reaction.

The levels of gene expressions of CK19, CK7, AQP1 and AE2 by mRNA quantification in cholangiocytes in culture were determined by amplifying specific fragments of each gene by conventional PCR using the corresponding cDNAs. The expression of the *GAPDH* gene was used as an internal expression control (housekeeping gene). The primers used to specifically amplify the markers mentioned above are shown in **Table 1.**

**Table 1.** Oligonucleotides used for the PCR reaction

| Gene | Sense sequence (5'→3') | Antisense sequence (5' → 3') |
|---|---|---|
| ***AE2*** | SEQ ID NO. 1 | SEQ ID NO. 2 |
| ***AQP1*** | SEQ ID NO. 3 | SEQ ID NO. 4 |
| ***CK19*** | SEQ ID NO. 5 | SEQ ID NO. 6 |
| ***CK7*** | SEQ ID NO. 7 | SEQ ID NO. 8 |
| ***GAPDH*** | SEQ ID NO. 9 | SEQ ID NO. 10 |

The results show that both normal and polycystic human cholangiocytes in culture express all these markers typical of cholangiocytes at the level of mRNA (Figure 1). Furthermore, whereas the expression levels of CK7, CK19 and AE2 were similar between the two cell types, the expression of AQP1 was higher in polycystic than in normal human cholangiocytes **(Figure 1),** suggesting high secretion by polycystic cells.

***Example** 2. **Polycystic human cholangiocytes** in **culture show an increase** in **basal metalloproteolytic activity compared to normal human cholangiocytes.***

Metalloproteolytic or metalloprotease activity was measured in supernatants of cultures of normal human cholangiocytes and polycystic human cholangiocytes by an assay based on the cleavage of the ES001 fluorogenic synthetic peptide (R&D Systems Minneapolis, MN, USA) that emits fluorescence proportional to metalloproteolytic activity. The substrate Mca-Pro-Leu-Gly-Leu-Dpa-Ala-Arg-$NH_2$ [Mca: (7-Methoxycoumarin-4-yl) acetyl, Dpa: N-3-(2, 4-Dinitrophenyl)-L-2,3-diaminopropionyl] (Knight CG, et al. FEBS Letters 1992;296:263) is lysed between glycine and leucine by the action of the MMPs, especially MMP-1 (collagenase 1), MMP-2 (gelatinase A), MMP-7 (matrilysin), MMP-8 (collagenase 2), MMP-9 (gelatinase B), MMP-12 (macrophage elastase), MMP-13 ( collagenase 3), MMP-14 (MT1-MMP), MMP-15 (MT2-MMP) and MMP-16 (MT3-MMP).

The basal metalloproteolytic activity of normal human cholangiocyte and polycystic human cholangiocytes cultures was determined by trypsinising the contents of 250 ml flasks and counting the number of cells in an inverted optical microscope using a Neubauer camera and a ½ dilution of the cell solution with *trypan blue* (Sigma). Next, 300,000 cells were seeded in each well of a 6-well plate and incubated with enriched DMEM/F-12 medium for 24 hours. After this time, the cells were washed with PBS and the culture medium changed for 2 ml of quiescent medium (DMEM/F-12, 1% FBS and 1% P/S) and incubated for 6 hours at 37 °C/5% $CO_2$. The supernatant was collected from each well and centrifuged at 4 °C for 10 minutes at 1,500 rpm to remove the dead cells present in the supernatant. The supernatant was concentrated 50 times using a 15 kDa (Millipore) Amicon Ultra concentrator. Next the concentrated supernatant was incubated for 2 hours at 37 °C with the ES001 fluorogenic peptide at a final concentration of 10 $\mu$M. Also, GM6001 (a global MMP inhibitor) and aprotinin (a global inhibitor of serine proteases) (both from Sigma-Aldrich) were used as controls of the specificity of the analysis of MMP activity in the assay. Recombinant MMP-10 (kindly provided by Dr. José Antonio Rodríguez García, Laboratorio de Ateroesclerosis e Inflamación del CIMA, Universidad de Navarra) was used as a positive control and DMEM/F-12 medium without serum was used as the negative control. The fluorescence determination was kinetically measured in a Spectra MAX GeminiXS(Molecular Devices Sunnyvale, CA, USA) spectrophotometer at excitation-emission wavelengths of 320-405 nm. The fluorescence intensity emitted by the culture medium was extracted from the metalloproteolytic activity values. The metalloproteolytic activity was calculated from the tangent to the curve of the fluorescence values using at least six different points over time for each type of cell and condition.

The results demonstrated that polycystic human cholangiocytes have higher basal metalloproteolytic activity compared to normal human cholangiocytes in culture ($p<0.001$; **Figure 2).** This effect was specific because the metalloproteolytic activity of both cell cultures was inhibited in the presence of the MMP inhibitor GM6001 ($p<0.001$ for both groups; **Figure 2).** In addition, recombinant MMP-10 used as a positive control increased the metalloproteolytic activity of the assay, and this was inhibited by GM6001 **(Figure 2).**

All the statistical calculations carried out for obtaining the data shown in the present invention were performed using the *GraphPad Prism* 5 (GraphPad Software) computer program. The normality of the data obtained was analysed using the D'Agostino-Pearson test. For comparison between 2 groups, the Student's t-test was used (when the distribution of the variable was normal) or the Mann-Whitney U test (when the distribution of the variable did not match the parameters of normality). For comparisons between three or more groups, a one-factor ANOVA test was performed followed by Bonferroni's a posteriori test. Differences were considered significant when $p<0.05$.

***Example 3. Polycystic human cholangiocyte cultures show changes in the pattern of expression of metalloproteinases and natural metalloprotease inhibitors at the level of mRNA compared to normal human cholangiocytes.***

To determine if the metalloproteolytic hyperactivity of polycystic human cholangiocytes is associated with changes in the pattern of expression of MMPs and their natural inhibitors, TIMPs, at the level of mRNA, this was analysed by quantitative PCR (i.e. qPCR) using the cDNA previously obtained as explained in Example 1, the gene expression of a total of 22 genes, including those containing the members of the metalloprotease family: MMP-1, MMP-2, MMP-3, MMP-7, MMP-8, MMP-9, MMP-10, MMP-11, MMP-13, MMP-14, MMP-15, MMP-16, MMP-17, MMP-18, MMP-24, MMP-25, MMP-26, MMP-27 and their natural inhibitors: TIMP-1, TIMP-2, TIMP-3, TIMP-4. Briefly, 4 $\mu$l of the cDNA were taken and 1.8 $\mu$l of a 10 $\mu$M solution of each primer (final concentration 0.9 $\mu$M) and 10 $\mu$l of *FastSYBR® Green* (Applied Biosystems) were added, adding sterile water (Braun) to reach a final volume of 20 $\mu$l. The primers used are shown in **Table 2.** The amplification reactions were performed in a *StepOne Plus* (Applied Biosystems) thermo cycler following the standard protocol conditions for *FastSYBR Green®.*

**Table 2.** Oligonucleotides used for quantitative PCR.

| Gene | Sense primer (5'→3') | Antisense primer (5' → 3') |
|---|---|---|
| ***MMP1*** | SEQ ID NO. 11 | SEQ ID NO. 12 |

(continued)

| Gene | Sense primer (5'→3') | Antisense primer (5' → 3') |
|---|---|---|
| ***MMP2*** | SEQ ID NO. 13 | SEQ ID NO. 14 |
| ***MMP3*** | SEQ ID NO. 15 | SEQ ID NO. 16 |
| ***MMP7*** | SEQ ID NO. 17 | SEQ ID NO. 18 |
| ***MMP8*** | SEQ ID NO. 19 | SEQ ID NO. 20 |
| ***MMP9*** | SEQ ID NO. 21 | SEQ ID NO. 22 |
| ***MMP10*** | SEQ ID NO. 23 | SEQ ID NO. 24 |
| ***MMP11*** | SEQ ID NO. 25 | SEQ ID NO. 26 |
| ***MMP13*** | SEQ ID NO. 27 | SEQ ID NO. 28 |
| ***MMP14*** | SEQ ID NO. 29 | SEQ ID NO. 30 |
| ***MMP15*** | SEQ ID NO. 31 | SEQ ID NO. 32 |
| ***MMP16*** | SEQ ID NO. 33 | SEQ ID NO. 34 |
| ***MMP17*** | SEQ ID NO. 35 | SEQ ID NO. 36 |
| ***MMP18*** | SEQ ID NO. 37 | SEQ ID NO. 38 |
| ***MMP24*** | SEQ ID NO. 39 | SEQ ID NO. 40 |
| ***MMP25*** | SEQ ID NO. 41 | SEQ ID NO. 42 |
| ***MMP26*** | SEQ ID NO. 43 | SEQ ID NO. 44 |
| ***MMP27*** | SEQ ID NO. 45 | SEQ ID NO. 46 |
| ***TIMP-1*** | SEQ ID NO. 47 | SEQ ID NO. 48 |
| ***TIMP-2*** | SEQ ID NO. 49 | SEQ ID NO. 50 |
| ***TIMP-3*** | SEQ ID NO. 51 | SEQ ID NO. 52 |
| ***TIMP-4*** | SEQ ID NO. 53 | SEQ ID NO. 54 |

The relative estimate of the mRNA levels of the genes of **Table 2** were calculated from the PCR progression curve (fluorescence vs. cycle), where the cycle threshold or Ct was determined from when the fluorescence signal begins to increase exponentially. Thus to calculate the difference of relative expression of the normal and polycystic human cholangiocytes, the data obtained were analysed following the modified method of Livak and Schmittgen (Livak KJ, et al. Methods 2001;25:402), according to the following equation:

$$\textit{Relative values} = 1.8\ (^{\text{polycystic or normal cholangiocyte GAPDH - polycystic or normal cholangiocyte GEN}})$$

Where the superscripts "*GAPDH* polycystic or normal cholangiocytes" and "*Gene* polycystic or normal cholangiocytes" correspond to the Ct for *GAPDH* and for each studied gene. The value of 1.8 (in place of 2) was used because it is assumed that the real efficiency of each amplification cycle is around 80% instead of 100%.

The results indicated that polycystic human cholangiocytes in culture basally show an altered expression pattern of metalloproteases at the mRNA level compared to normal human cholangiocytes in culture. These data were grouped according to the structure and function of each type of metalloprotease into: collagenases, gelatinases, stromelysins, matrilysins and membrane metalloproteases.

Analysis of the expression levels of the MMPs with collagenase activity (i.e. characterised by degrading fibrillar collagen: collagen type I, II, III and VII) showed that polycystic human cholangiocytes in culture exhibited an increase in the expression of MMPs 1 and 18 (~4 and -12 times respectively) compared to normal human cholangiocytes in culture (p<0.01 in both groups; **Figure 3).** By contrast, changes in the expression of MMP-8 were not detected between the two cell types and the level of expression of MMP-13 was reduced in the polycystic human cholangiocytes in culture compared to the normal human cholangiocytes (p<0.05; **Figure 3).**

Analysis of the mRNA expression levels of the MMPs with gelatinase activity, which are involved in the degradation of denatured interstitial collagen (i.e. collagen type IV and V of the basal membrane), showed that MMP-2 was increased ~3 times (p<0.01; **Figure 4)** in polycystic human cholangiocytes compared to normal human cholangiocytes in culture. However, the expression levels of MMP-9 were reduced in polycystic human cholangiocytes compared to normal human cholangiocytes in culture (p<0.01; **Figure 4)**

The study of the mRNA expression levels of the MMPs with stromelysin activity, which participate in the degradation of a large number of substrates (i.e. collagen, fibronectin, laminin, gelatin, casein and a large number of MMPs) revealed that MMPs 3, 10 and 11 were increased by ~6, ~2 and -1.5 times in polycystic human cholangiocytes compared to normal human cholangiocytes in culture (p<0.01; for all the groups; **Figure 5).** On the other hand, expression levels of MMP-27 were unchanged between both cell types **(Figure 5).**

The matrilysins are the simplest group of MMPs because they do not contain the homologous hemopexin domain, being more susceptible to degradation or inhibition by the TIMPs. Analysis of the mRNA expression levels of MMP-7 showed that these values were reduced in polycystic human cholangiocytes compared to normal human cholangiocytes in culture (p<0.01; **Figure 6).**

The membrane metalloproteases (i.e. MMPs 14, 15, 16, 17, 24 and 25) are very important because of their ability to bind to the cell membrane and intervene in the proteolytic activation of other metalloproteases. The results reveal that the mRNA expression levels of MMPs 14, 16, 17 and 25 were increased by ~3, ~5, ~3 and ~4 times respectively in polycystic human cholangiocytes compared to normal human cholangiocytes in culture (p<0.01; for all; **Figure 7).** By contrast, the mRNA expression levels of MMP-15 and MMP-24 were reduced in polycystic human cholangiocytes compared to normal human cholangiocytes in culture (p<0.01; **Figure 7).**

Therefore the results demonstrate that the mRNA expression levels of the MMPs 1, 2, 3, 10, 11, 14, 16, 17, 18 and 25 were increased in polycystic human cholangiocytes compared to normal human cholangiocytes in culture. On the other hand, the mRNA expression levels of the MMPs 7, 9, 13, 15 and 24 were reduced in polycystic human cholangiocytes compared to normal human cholangiocytes in culture; it was similarly shown that the MMPs 8 and 27 were equally expressed in both types of cells.

The metalloproteolytic activity of the metalloproteases in the extracellular space is regulated by activation and inhibition mechanisms. Thus MMP inhibition is mediated by endogenous inhibitors known as the tissue inhibitors of metalloproteinases (TIMPs). The TIMPs exercise their inhibitory function by forming a reversible high affinity complex with the metalloproteases. Analysis of the gene expression levels of the various TIMPs (i.e. TIMP-1 to -4) at the level of mRNA revealed an increase in TIMPS 1, 2 and 3 (by ~5, ~2 and ~5 times respectively) in polycystic human cholangiocytes compared to normal human cholangiocytes in culture (p<0.01 for all groups; **Figure 8).**

By contrast, the mRNA expression levels of TIMP-4 were reduced in polycystic human cholangiocytes compared to normal human cholangiocytes in culture (p<0.01; **Figure 8).** This was because: i) the basal metalloproteolytic activity of polycystic human cholangiocytes is increased compared to that of normal human cholangiocytes and ii) there is an increased global expression pattern at the basal level of the various metalloproteases in polycystic human cholangiocytes, the over-expression of TIMP-1, -2 and -3 could be a compensatory mechanism that uses polycystic cholangiocytes to attempt to reduce this metalloproteolytic hyperactivity, although this is ultimately inefficient.

***<u>Example 4.</u>* *Interleukins* 6 *and* 8, *and also the oestrogen 17β-estradiol, increase metalloproteolytic activity of normal human cholangiocytes and polycystic human cholangiocytes in culture.***

Next, the role of hormones, cytokines and growth factors, present in the cystic liquid of patients with PLDs, on the metalloproteolytic activity shown by normal and polycystic human cholangiocytes was evaluated. Therefore, the effect of the cytokines IL-6 and IL-8, of oestrogen 17β-estradiol (17β-Es), and of the growth factors vascular endothelial growth factor (VEGF), hepatocyte growth factor (HGF), epidermal growth factor (EGF), growth-regulated oncogene α (GROα) and epithelial neutrophil-activating peptide 78 (ENA-78), all present at high concentrations in the cystic fluid (Amura CR, et al. Am J Physiol Cell Physiol 2008;294:C786-796; Nichols MT, et al. Hepatology 2004;40:836) were investigated.

To analyse the effect of various stimuli on the metalloproteolytic activity of normal and polycystic human cholangiocytes cultures, a protocol similar to that described before in Example 2 was followed, but in this case the incubation of the cells was with quiescent medium (i.e. DMEM/F12, 3% FBS, 5% penicillin-streptomycin) in the presence of the corresponding stimulus **(Table 3).** Next, the same procedure as described for the analysis of basal metalloproteolytic activity was followed as described in Example 2.

**Table 3.** Stimuli used for the analysis of metalloproteolytic activity.

| Treatments | Final Concentration | Company |
|---|---|---|
| VEGF | 10 ng/ml | R&D Systems |

(continued)

| Treatments | Final Concentration | Company |
|---|---|---|
| EGF | 10 ng/ml | R&D Systems |
| IGF 1 | 10 ng/ml | R&D Systems |
| TGFB | 0.08 ng/ml | R&D Systems |
| HGF | 20 ng/ml | R&D Systems |
| IL-8 | 50 ng/ml | PeproTech |
| IL-6 | 50 ng/ml | PeproTech |
| ENA-78 | 10 ng/ml | PeproTech |
| GRO-α | 10 ng/ml | PeproTech |
| 17β-estradiol | 1 nM/ml | Steraloids |

The results demonstrated that interleukins 6 and 8, and also 17β-Es, cause an increase in metalloproteolytic activity in both normal and polycystic human cholangiocytes compared to both cell types in the basal situation **(Figure 9).** These increases in proteolytic activity were similar between normal and polycystic human cholangiocytes **(Figure 9).** By contrast, the growth factors (i.e. VEGF, HGF, EGF, ENA-78 and GROα) did not induce any change in metalloproteolytic activity of either cell type in the experimental conditions used **(Figure 9).**

***Example 5**. Interleukins 6 and 8 and also oestrogen 17β- estradiol, increase the expression of various metalloproteases in both normal and polycystic human cholangiocytes in culture.*

Taking into account the increase in metalloproteolytic activity induced by interleukins 6 and 8 and also the oestrogen 17β-estradiol in human cholangiocytes in culture, this was further investigated to determine if the increase in activity was correlated with an increase in gene expression at the level of mRNA of the various MMPs.

The results obtained indicated that the expression of MMP-2 was increased in polycystic human cholangiocytes in the presence of IL-6 and of 17β-estradiol ($p<0.05$ in both groups; **Figure 10),** whereas its expression was not changed by IL-8 **(Figure 10).** By contrast, none of these modules induced changes in MMP-2 expression in normal human cholangiocytes **(Figure 10).**

The expression levels of MMP-3 increased in the presence of IL-6 in polycystic human cholangiocytes in culture ($p<0.05$; **Figure 11**), whereas its expression was unchanged by IL-8 and 17β-estradiol. The expression levels of MMP-3 increased in normal human cholangiocytes in culture in the presence of 17β-estradiol ($p<0.05$; **Figure 11**), whereas its expression was unchanged by IL-6 and IL-8.

The expression of MMP-7 increased in the presence of IL-6 in polycystic human cholangiocytes in culture ($p<0.05$; **Figure 12),** whereas its expression was unchanged by IL-8 and 17β-estradiol. None of the 3 molecules caused changes in expression of MMP-7 in normal human cholangiocytes in culture **(Figure 12).**

The expression of MMP-10 increased in the presence of IL-8 and 17β-estradiol in normal human cholangiocytes in culture ($p<0.05$ and $p<0.01$ respectively; **Figure 13),** whereas its expression was unchanged by IL-6. By contrast, none of the 3 molecules caused changes in MMP-10 expression in polycystic human cholangiocytes in culture **(Figure 13).**

The expression of MMP-11 increased in both normal and polycystic human cholangiocytes in the presence of IL-8 or 17β-estradiol ($p<0.05$ and $p<0.01$ respectively for both cell types; **Figure 14).** IL-6 increased MMP-11 expression in polycystic human cholangiocytes but did not change MMP-11 expression in normal human cholangiocytes in culture **(Figure 14).**

The expression of MMP-13 increased in both normal and polycystic human cholangiocytes in culture in the presence of 17β-estradiol ($p<0.01$ for both cell types; **Figure 15).** IL-8 increased MMP-13 expression in normal human cholangiocytes ($p<0.05$ **Figure 15),** but did not change its expression in polycystic human cholangiocytes **(Figure** 15). Also, IL-6 did not cause changes in MMP-13 expression in any of the cell types.

The expression of MMP-15 increased in polycystic human cholangiocytes in culture in the presence of IL-6 ($p<0.05$; **Figure 16),** whereas its expression was not altered by IL-8 or 17β-estradiol. These molecules did not change the expression of MMP-15 in normal human cholangiocytes **(Figure 16).**

The expression of MMP-17 increased in polycystic human cholangiocytes in the presence of IL-6 ($p<0.01$; **Figure 17),** IL-8 and 17β-estradiol ($p<0.05$ for both groups; **Figure 17).** By contrast, these 3 molecules did not change the expression of this MMP in normal human cholangiocytes in culture **(Figure 17).**

Finally, the expression of MMP-27 increased in polycystic human cholangiocytes in the presence of IL-6 ($p<0.01$; **Figure 18),** whereas its expression was unchanged by IL-8 and 17β-estradiol. Furthermore, these 3 molecules did not change the expression of this MMP in normal human cholangiocytes in culture **(Figure 18).**

The other metalloproteinases 1, 8, 9, 14, 16, 18, 24 and 26 did not show changes in expression in normal and polycystic human cholangiocytes in the presence of IL-6, IL-8 or 17β-estradiol.

*__Example__ 6. **The presence of anti-IL6R and anti-CXCR1 antibodies reduces basal metalloproteolytic hyperactivity of polycystic human cholangiocytes in culture.***

Taking into account that: i) basal activity of polycystic human cholangiocytes in culture is greater than that of normal human cholangiocytes and ii) that IL-6 and IL-8 increased the metalloproteolytic activity of normal and polycystic human cholangiocytes, the role of these two cytokines on metalloproteolytic hyperactivity of polycystic human cholangiocytes using antibodies against the receptors of IL-6 (IL6R) and/or IL-8 (CXCR1) was investigated. The protocol was similar to the analysis of metalloproteolytic activity after addition of stimuli, but in this case the cells were incubated with neutralising antibodies at a final concentration of 0.3 μm/ml: Monoclonal Anti-IL-6R-alpha (Millipore), Monoclonal Anti-CXCR1 (IL-8R) (Abcam) and goat anti-rabbit (Thermo Scientific) in quiescent medium (i.e. DMEM/F12, 3% FBS, 5% penicillin-streptomycin). In addition, in both cell types a negative control using secondary antibodies of goat that recognise rabbit IgGs (goat anti-rabbit; Roche) was used.

The presence of antibodies against IL6R in the culture medium caused a decrease in metalloproteolytic activity in polycystic human cholangiocytes compared to this cell type incubated with the negative control antibody ($p<0.001$; **Figure 19).** Also, incubation of polycystic human cholangiocytes with antibodies against the IL-8 receptor (CXCR1) caused a decrease in the basal metalloproteolytic hyperactivity compared to this cell type in the presence of negative control antibodies ($p<0.001$; **Figure 19).** Co-incubation of anti-IL6R and anti-CXCR1 antibodies in polycystic human cholangiocytes did not induce a synergic effect on the decrease of metalloproteolytic activity ($p<0.05$; **Figure 19).** This could be the consequence of both cytokines regulating common signalling pathways that result in an increase in general metalloproteolytic activity.

In addition, metalloproteolytic activity of normal human cholangiocytes in culture was analysed in the presence of anti-IL6R and/or anti-CXCR1 antibodies. The results show that the presence of these antibodies did not cause any change in metalloproteolytic activity of normal human cholangiocytes compared to this cell type incubated with negative control antibody **(Figure 20).**

*__Example 7__. **Marimastat and 13-cis-retinoic acid reduce metalloproteolytic activity of normal and polycystic human cholangiocytes in culture.***

The metalloproteolytic activity of normal and polycystic human cholangiocytes in culture was determined in the presence of two types of inhibitors: Marimastat, a pseudopeptide inhibitor that binds to the catalytic site of MMPs by its chelating action on zinc, and 13-cis-retinoic acid (R&D Systems) that regulates the expression of the metalloproteases through its binding to its promoter AP-1. A similar protocol to that described in the previous section was followed, but in this case the cells were incubated in quiescent medium (i.e. DMEM/F12, 3% FBS, 5% penicillin-streptomycin) in the presence of these inhibitors: 13-cis-retinoic acid (10 μM/ml) or Marimastat (200 μM/ml).

Treatment of cultures of normal human cholangiocytes and polycystic human cholangiocytes with 13-cis-retinoic acid or Marimastat inhibited the basal metalloproteolytic activity of both cells ($p<0.01$; **Figure 21).**

*__Example__ 8. **Expression of MMPs -2 and -3 is increased in cholangiocytes of patients with PLDs.***

The expression of MMPs -1, -2 and -3 in the following tissue types was analysed by immunohistochemical techniques: i) liver of normal individuals, ii) liver of ARPKD patients, iii) liver of ADPKD patients, iv) liver of normal rats and v) liver of PCK rats.

PCK rats, sold by the Charles River company, is a well characterised animal model of ARPKD and used for the study of liver and kidney cystogenesis (Mason SB, et al. The Anatomical Record: Advances in Integrative Anatomy and Evolutionary Biology 2010;293:1279-1288). This colony of animals has a spontaneous orthologous mutation in the *PKHD1* gene that causes them to develop liver and kidney symptoms similar to those of ARPKD patients. Thus, after 4 weeks of life, PCK rats, irrespective of sex, start to develop liver and kidney cystogenesis and fibrosis, which are clearly shown at 16 weeks of life. In addition, these animals also show an increase in serum values of creatinine, bilirubin, cholesterol, triglycerides and proteinuria (Mason SB, et al. The Anatomical Record: Advances in Integrative Anatomy and Evolutionary Biology 2010;293:1279-1288). PCK rats were maintained on standard diet for the whole assay and all *in vivo* experimental procedures were approved by the Ethics Committee of the Instituto Biodonostia.

Slices of liver tissue included in paraffin were deparaffinised and hydrated by placing them in a series of ethanol

solutions of decreasing ethanol concentration (100%, 70% and 50%) and finally in phosphate buffer (PBS-1X). After the tissue was hydrated and in order to unmask the antigens, the slices were incubated at 95 °C for 20 minutes in a solution of Tris-EDTA at pH 9. Before incubation with specific antibodies, endogenous peroxidase was blocked with a hydrogen peroxide solution (diluted in water to 30%) for 10 minutes (Panrea Química SU). Next, the slices were washed 3 times for 5 minutes with distilled water and finally with PBS-1X. In order to prevent non-specific binding of immunoglobulins (IgGs), the tissues were incubated for 1 hour with normal goat serum diluted to 5% in PBS.

After completion of the previous step, the slices of liver tissue were incubated with specific polyclonal antibodies against each type of MMP, which were diluted 1:50 in the same blocking solution used in the previous stage and were kept in a humidified chamber at 4 °C overnight. The antibodies used were the anti-MMP-1 (Aviva System Biology), anti-MMP-2 and anti-MMP-3 (Abcam) antibodies. On the following day, the samples were incubated with goat anti-rabbit-HRP EnVision TM (DakoCytomation) secondary antibodies for 1 hour at room temperature. Next, after the washes, they were incubated with the chromogen DAB (DakoCytomation) for 10 minutes at room temperature and then they were counterstained with hematoxylin (Mayer). To finish, the samples were dehydrated in baths of increasing concentrations of ethanol (50%, 70% and 100%) and were mounted using DePeX mounting medium (Sigma) for subsequent viewing in a Nikon optical microscope (Eclipse E800).

The results obtained through these assays indicated that the expression of the MMPs -2 and -3 were increased in polycystic cholangiocytes of patients with ARPKD and ADPKD compared to cholangiocytes of normal individuals **(Figure 22).** Thus the expression of MMPs -2 and -3 was also increased in polycystic cholangiocytes of PCK rats compared to cholangiocytes of normal rats **(Figure 23).** These proteic over-expressions were mainly located in the cytoplasm of polycystic cholangiocytes. Proteic expression of MMP-1 was not detected in normal or polycystic liver tissue; furthermore, the expression of MMP-1 was also not observed in the liver tissue of normal or PCK rats.

***Example 9. Treatment of PCK rats with Marimastat inhibits liver cystogenesis***

To further investigate the action of the metalloproteases in liver cystogenesis, the possible therapeutic role of Marimastat was investigated, inhibiting liver cystogenesis, as well as blocking the fibrogenic processes that develop chronically in PCK rats through the inhibition of metalloproteolytic hyperactivity of polycystic cholangiocytes.

Male PCK rats of 8 weeks of age were divided into 2 groups: i) control (placebo), and ii) treated with Marimastat. Eleven (11) animals were used in the control group and ten (10) in the group treated with Marimastat (initial weight -250 g). Marimastat was administered daily (2 times per day every ~ 8-12 hours) orally using a orogastric tube. Marimastat (Tocris Biosciences, reference 2631) was administered at a dose of 0.2856 mg/kg/day (dose used in clinical trials in patients with colorectal cancer that has beneficial effects and does not cause toxicity) (Nemunaitis J, et al. Clinical Cancer Research 1998;4:1101-1109) dissolved in distilled water (Fresenius Kabi). The values of Marimastat given were corrected with the weight of the animals (which was monitored daily). The group of control rats received the vehicle solution (i.e. distilled water) orally, 2 times a day (every ~ 8-12 hours) and using the tube. Various biochemical markers were monitored in serum at the start and end of the study. Blood was extracted (500 μl through the tail vein) after anaesthetising the animal with isoflurane (inhalation anaesthesia, 2.5% in oxygen, with a flow rate of 0.3 L/min). After centrifuging the blood, various biochemical parameters were determined in the serum: alkaline phosphatase (ALP), transaminases (ALT, AST), bilirubin, biliary acids, creatinine, cholesterol, triglycerides and total proteins using a COBAS Integra400 Plus 174 analyser. The rats of both groups (control and treated with Marimastat) were sacrificed at 8 weeks of treatment.

In addition, as a control, normal rats of 8 weeks of age were also treated with Marimastat orally for 1 week at the same dose as the rest of the animals. The various biochemical and histological markers listed above were also analysed. This assay showed that treatment of control rats with Marimastat did not affect either the histology of the liver or the kidney or the serum levels of biochemical markers in normal rats. There were also no changes in the mean body weight of the animals.

After sacrificing the animals, the liver and kidney were removed and weighed; next both types of tissue were sectioned with a scalpel and fixed with a solution of 10% formaldehyde, to be later included in paraffin. Slices of 5 μm thickness were made of each sample and stained with hematoxylin-eosin or picrosirius red.

The aim of hematoxylin-eosin staining is the determination of histological morphology. Thus the slices were incubated for 4 minutes with Harris hematoxylin (Merck) at room temperature. After incubation, the samples were washed with distilled water and stained with eosin (Merck) for 1 minute at room temperature. Next, the tissue slices were washed in running water for 5 minutes, dehydrated by immersion in solutions of increasing ethanol concentration (70%, 96%, 100% for 1 minute each) and finally incubated with xylol for 5 minutes. The samples were mounted with DePeX (Sigma) mounting medium for subsequent viewing in an optical microscope (Nikon Eclipse E800).

The purpose of picrosirius red staining is the determination of extracellular collagen in the tissue. When hydration was completed, the slices were stained with 0.2% phosphomolybdic acid (Panreac Química) for 2 minutes and washed with distilled water. Next, the samples were incubated with a solution of 0.1% picrosirius red f3B (Gibco) for 3 hours. Finally the samples were washed with a solution of 0.01 N HCl for 2 minutes; the tissue was then dehydrated and

mounted as described in the previous section.

Sacrificing the animals revealed that rats treated with Marimastat showed a reduction in liver cystogenesis (i.e. area of intrahepatic biliary cysts) compared to control PCK rats ($p<0.05$; **Figure 24).** Additionally, the level of expression of various profibrotic hepatic markers [collagen 1a1 (*Col1a1*), beta transforming growth factor *(TGFbeta),* alpha actin of smooth muscle *(alpha-Sma)* and the growth factor of connective tissue *(Ctgf)]* and proinflammatory markers *[IL-6, IL CXCL1* and *IL1beta*)] were analysed by RT-PCR in liver samples of control and PCK rats. The primers used are shown in **Table 6.** The fact that rats treated with Marimastat showed a reduction in liver cystogenesis compared to control PCK rats is associated with down regulation of cholangiocyte markers Ck19 ($p<0.001$; **Figure 27A)** and of collagen 1a1 (Col1a1) ($p<0.05$; **Figure 27B).** The PCK rats treated with Marimastat also showed an almost statistically significant down regulation of the mRNA levels of IL6 and CXCL1 ($p = 0.1$ and $p = 0.08$; **Figure 28A** and **B** respectively).

It is important to take into account that kidney cystogenesis was not affected by treatment with Marimastat **(Figure 25).**

The liver of PCK rats treated with Marimastat showed less collagen deposition compared to control PCK rats **(Figure 26),** whereas collagen deposition in kidney was similar in both groups.

Finally it should be highlighted that treatment with Marimastat did not change the weight of the animals nor of their tissues (liver and kidney) at the time of sacrifice **(Table 4).**

**Table 4.** Weight of animals and their organs in the two experimental groups of PCK rats, control and treated with Marimastat, at the time of sacrifice.

| PARAMETERS | | PCK Control (n=11) | PCK Marimastat (n=9) |
|---|---|---|---|
| WEIGHT | Rat | 507.59 ± 5.08 | 497 ± 12.24 |
| | Liver | 28.21 ± 3.45 | 26.17 ± 2.98 |
| | Left kidney | 2.18 ± 0.10 | 2.40 ± 0.19 |
| | Right kidney | 2.30 ± 0.15 | 2.36 ± 0.17 |

In addition, the serum levels of the various biochemical markers analysed (i.e. bicarbonate, alkaline phosphatase, AST, ALT, total bilirubin, glucose, total proteins, urate, urea, calcium, triglycerides, HDL, LDL, LDH and albumin) and biliary flow also showed no difference between the two study groups at the time of sacrifice **(Table 5).**

**Table 5.** Values of biliary flow and serum levels of biochemical markers of the two experimental groups of PCK rats, control and treated with Marimastat, at the time of sacrifice.

| PARAMETERS | PCK Control (n=11) | PCK Marimastat (n=9) |
|---|---|---|
| Biliary flow (uL/min/g liver) | 0.634 ± 0.10 | 0.49 ±0.10 |
| Bicarbonate (nmol/L) | 9.9 ± 0.33 | 9.26 ± 0.39 |
| Albumin (g/L) | 13.4 ±0.50 | 13.78 ± 0.59 |
| Creatinine (mg/dL) | 0.34 ± 0.02 | 0.36 ±0.02 |
| Glucose (mg/dL) | 154.7 ± 8.34 | 137.89 ± 7.25 |
| Triglycerides (mg/dl) | 66.8 ± 4.46 | 60.33 ± 3.97 |
| LDL (mg/dL) | 34.5 ± 2.58 | 33.38 ± 1.61 |
| HDL (mg/dL) | 24.5 ± 2.51 | 31.11 ± 3.06 |
| LDH (U/L) | 15111.1 ± 526.12 | 15192.33 ± 356.84 |
| Urea (mg/dL) | 20.5 ± 0.47 | 20.33 ± 0.78 |
| Total protein (g/dL) | 3.2 ± 0.14 | 3.11 ± 0.12 |
| Calcium (mg/dL) | 2.8 ± 0.07 | 2.70 ± 0.03 |
| Cholesterol (mg/dL) | 72.4 ± 4.20 | 76.56 ± 3.93 |
| Total bilirubin (mg/dL) | 0.11 ± 0.01 | 0.16 ± 0.05 |
| ALT (U/L) | 139.7 ± 4.07 | 138.22 ± 2.66 |

(continued)

| PARAMETERS | PCK Control (n=11) | PCK Marimastat (n=9) |
|---|---|---|
| AST (U/L) | 793.4 ± 41.34 | 755.56 ± 24.76 |
| Alkaline phosphatase (U/L) | 60.5 ±7.25 | 57.00 ± 5.01 |

**Table 6.** Oligonucleotides used in quantitative PCR for the analysis of profibrotic and proinflammatory markers in the animal model of control and PCK rats.

| Gene | Sense primer (5'→3') | Antisense primer (5' → 3') |
|---|---|---|
| Col1a1 | SEQ ID NO. 55 | SEQ ID NO. 56 |
| α-Sma | SEQ ID NO. 57 | SEQ ID NO. 58 |
| Tgfβ1 | SEQ ID NO. 59 | SEQ ID NO. 60 |
| Ctgf | SEQ ID NO. 61 | SEQ ID NO. 62 |
| IL6 | SEQ ID NO. 63 | SEQ ID NO. 64 |
| CXCL1 | SEQ ID NO. 65 | SEQ ID NO. 66 |
| IL1β | SEQ ID NO. 67 | SEQ ID NO. 68 |
| GAPDH | SEQ ID NO. 69 | SEQ ID NO. 70 |

All the results, both *in vitro* and *in vivo* demonstrate the effectiveness of Marimastat in the treatment of PLDs, inhibiting liver cystogenesis by blocking metalloproteolytic hyperactivity of polycystic cholangiocytes.

## Claims

1. Metalloprotease inhibitor (MMP) comprising a zinc binding group in its structure for use in the treatment of polycystic liver diseases (PLDs).

2. Metalloprotease inhibitor for use according to claim 1 wherein the metalloprotease inhibitor is selected from: pseudopeptide inhibitors, non-peptide inhibitors, tetracycline derivatives and/or bisphophonates.

3. Metalloprotease inhibitor for use according to any of claims 1-2 wherein the pseudopeptide inhibitor is selected from any of the list: marimastat and/or retinoic acid or any of their analogues.

4. Metalloprotease inhibitor for use according to any of claims 1-3 wherein the inhibitor is marimastat.

5. Metalloprotease inhibitor for use according to any of claims 1-4 wherein the non-peptide inhibitor is selected from any of the list: prinomastat, BMS-275291 and/or MMI270.

6. Pharmaceutical composition comprising at least one metalloprotease inhibitor comprising a zinc binding group in its structure, and pharmaceutically acceptable vehicles or excipients.

7. Pharmaceutical composition of claim 6 which further comprises another active principle.

8. Pharmaceutical composition of any of the claims 6-7 wherein the metalloprotease inhibitor is selected from: pseudopeptide inhibitors, non-peptide inhibitors, tetracycline derivatives and/or bisphophonates.

9. Pharmaceutical composition of any of the claims 6-8 wherein the pseudopeptide inhibitor is selected from: marimastat and/or retinoic acid or any of their analogues.

10. Pharmaceutical composition of the claims 6-9 wherein the pseudopeptide inhibitor is marimastat.

11. Pharmaceutical composition of any of the claims 6-10 wherein the non-peptide inhibitor is selected from: prinomastat, BMS-275291 and/or MMI270.

12. Pharmaceutical composition of any of the claims 6-11 wherein the active principle is selected from: somatostatin and/or any of its analogues and/or ursodeoxycholic acid.

13. Pharmaceutical composition of claim 12 wherein the somatostatin analogues are selected from: octreotide, lanreotide, vapreotide and/or pasireotide.

14. Pharmaceutical composition according to any of claims 6-13 for use in the treatment of polycystic liver diseases (PLDs).

# Figure 1

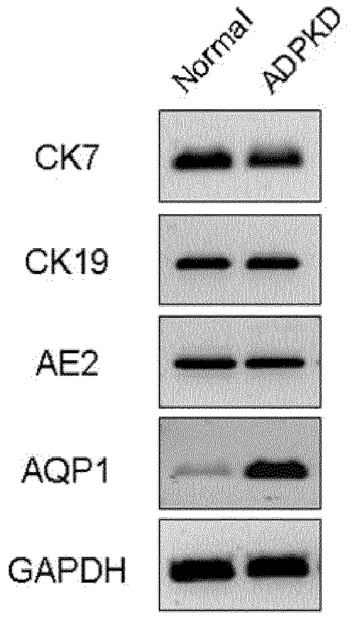


# Figure 2

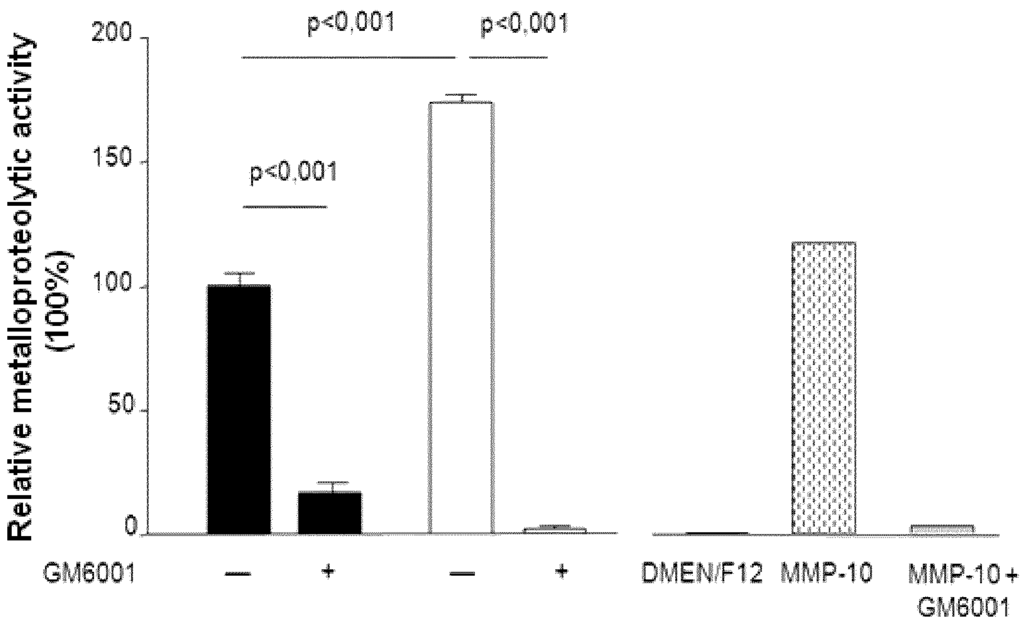

## Figure 3

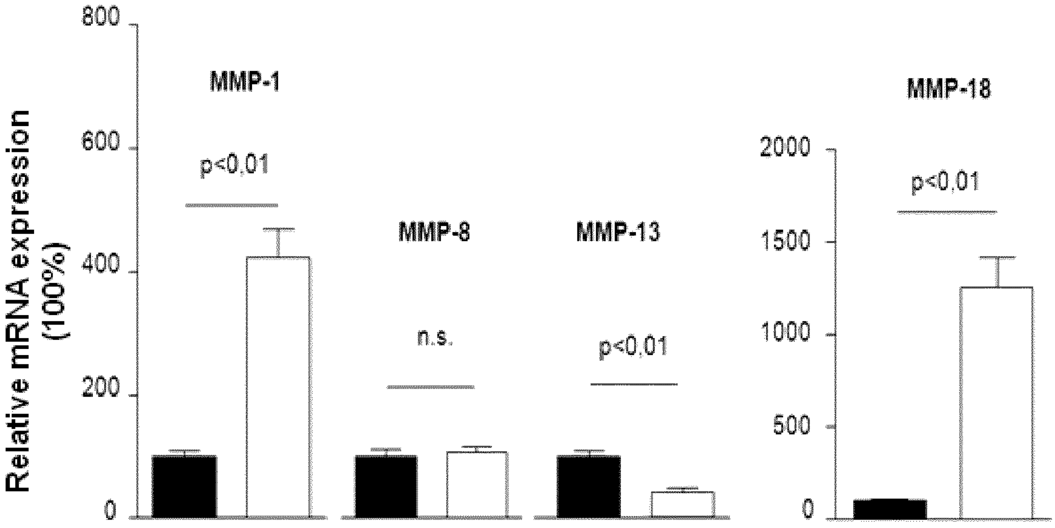


## Figure 4

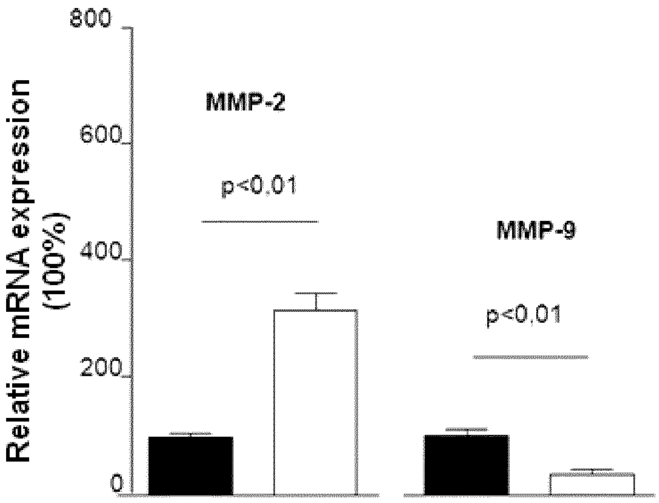

Figure 5

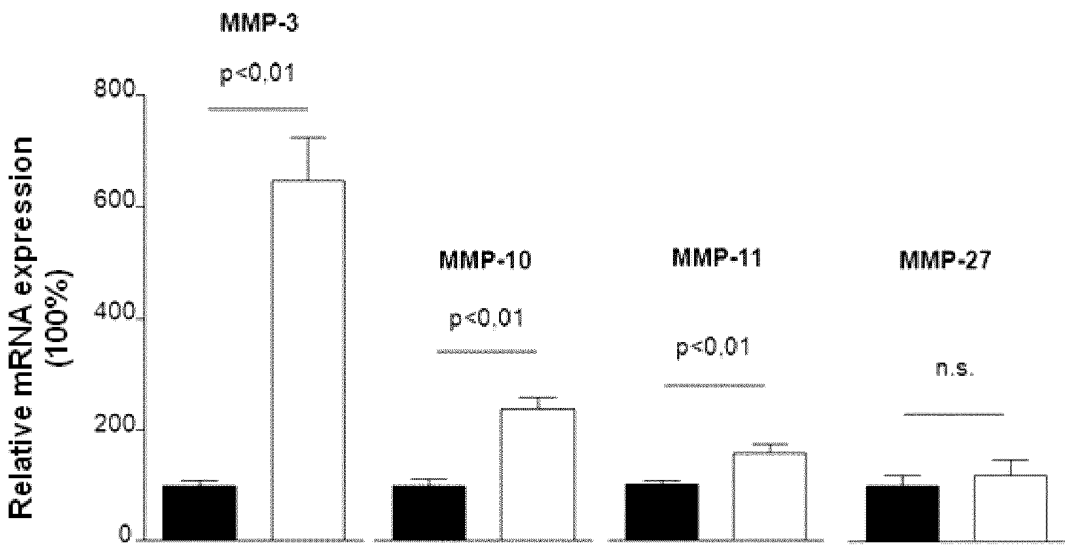


Figure 6

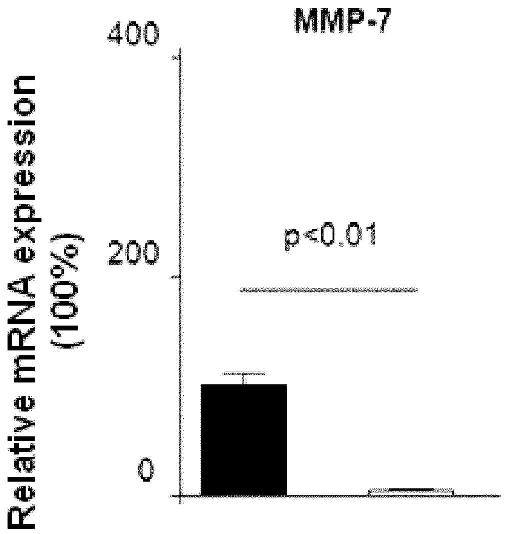

## Figure 7

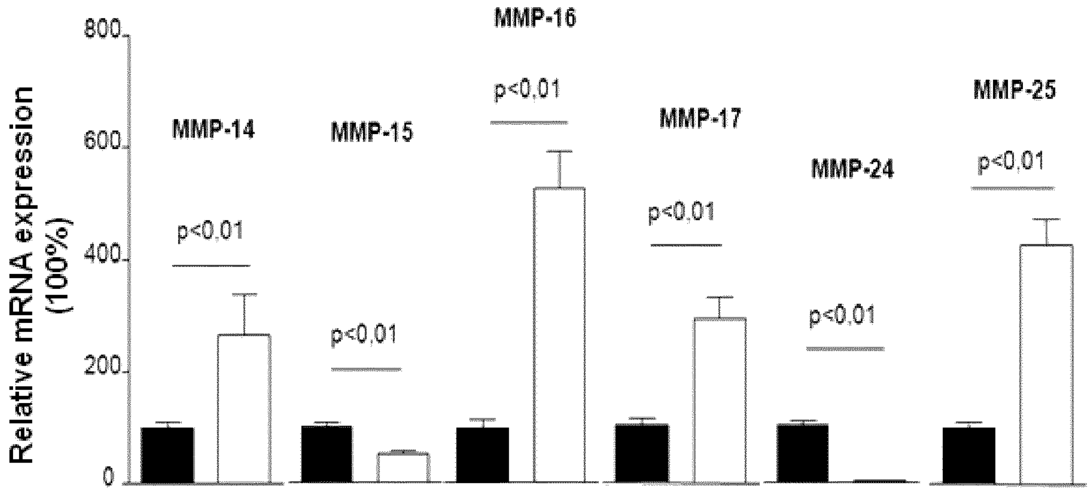


## Figure 8

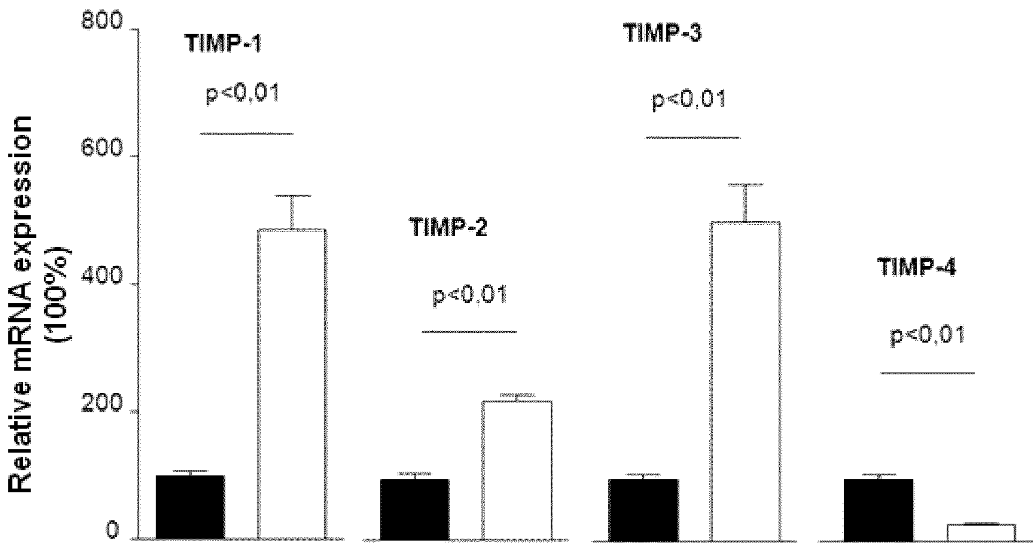

# Figure 9

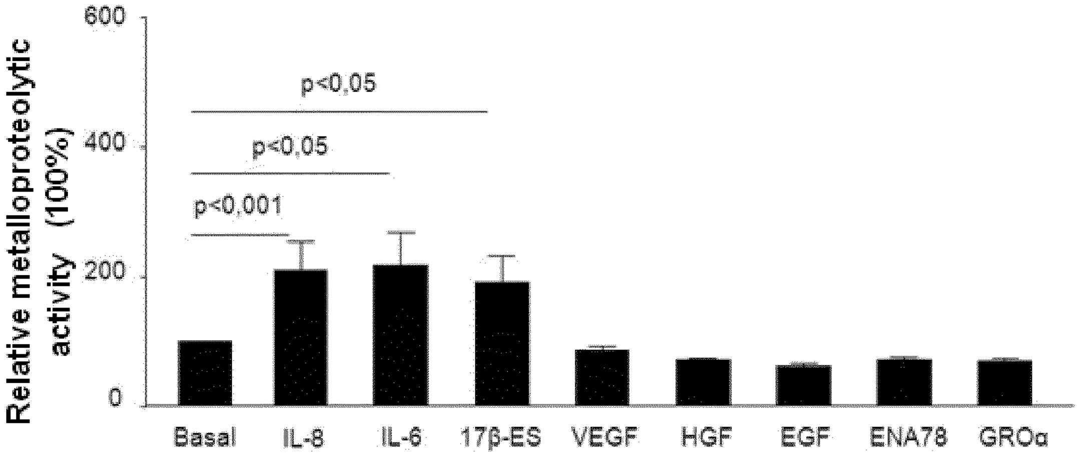
600
400
200
0
Relative metalloproteolytic activity (100%)
p<0,05
p<0,05
p<0,001
Basal
IL-8
IL-6
17β-ES
VEGF
HGF
EGF
ENA78
GROα

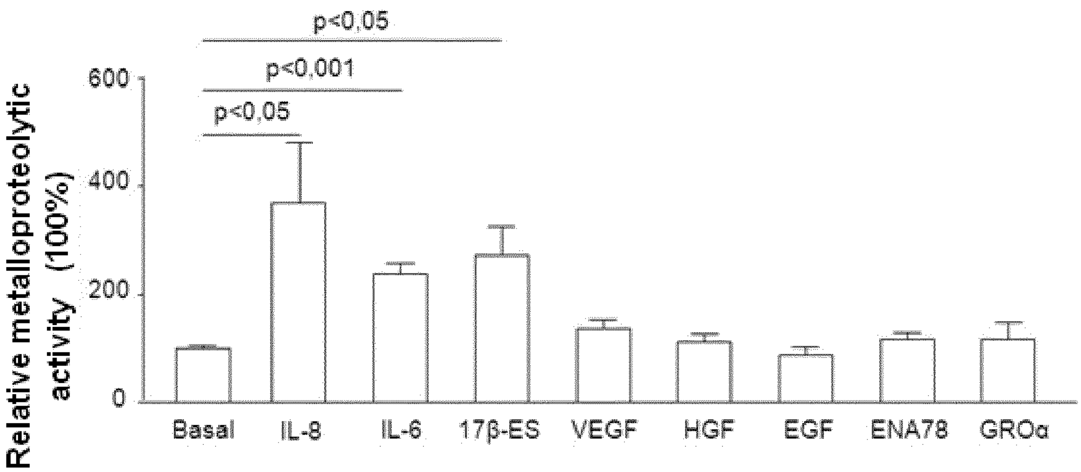
p<0,05
p<0,001
p<0,05
600
400
200
0
Relative metalloproteolytic activity (100%)
Basal
IL-8
IL-6
17β-ES
VEGF
HGF
EGF
ENA78
GROα

## Figure 10

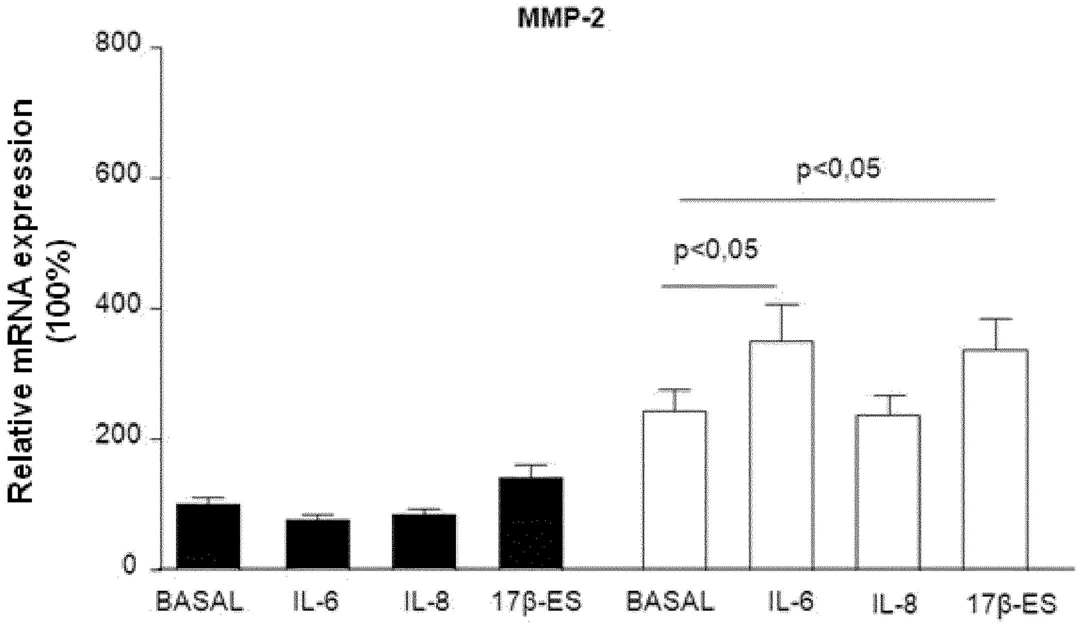


## Figure 11

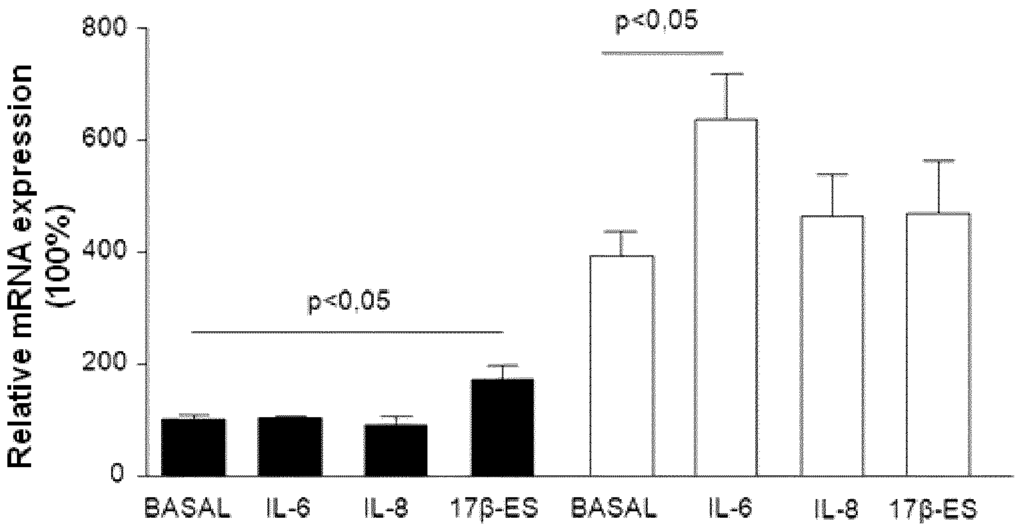

# Figure 12

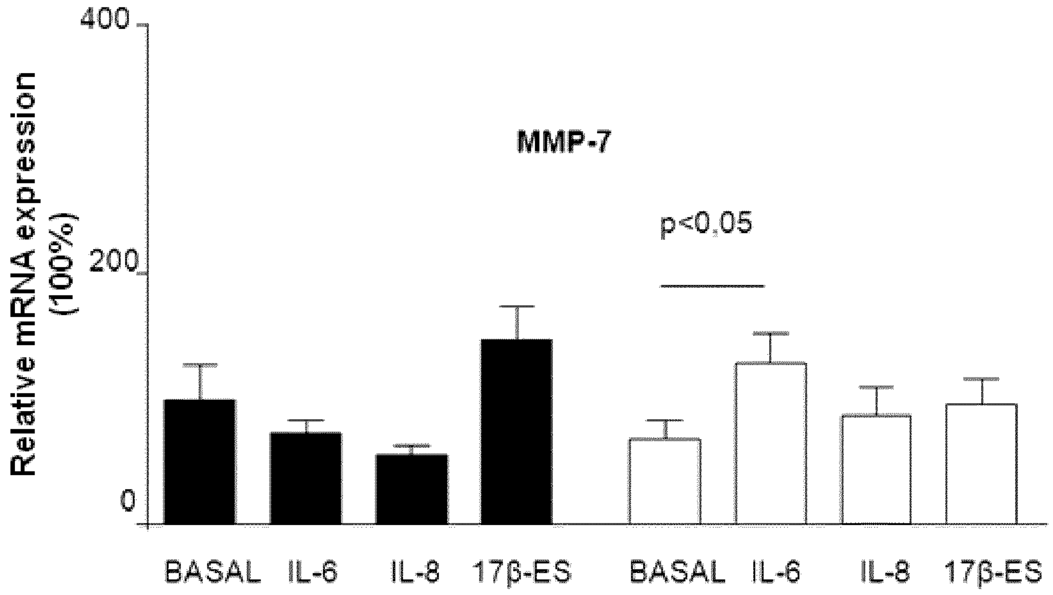


# Figure 13

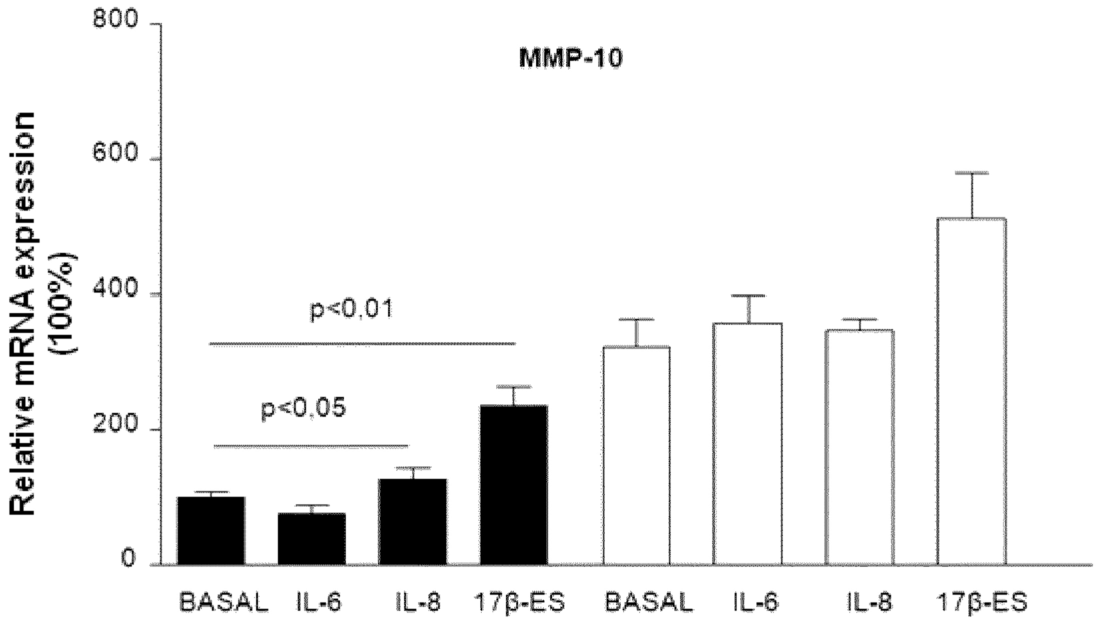

## Figure 14

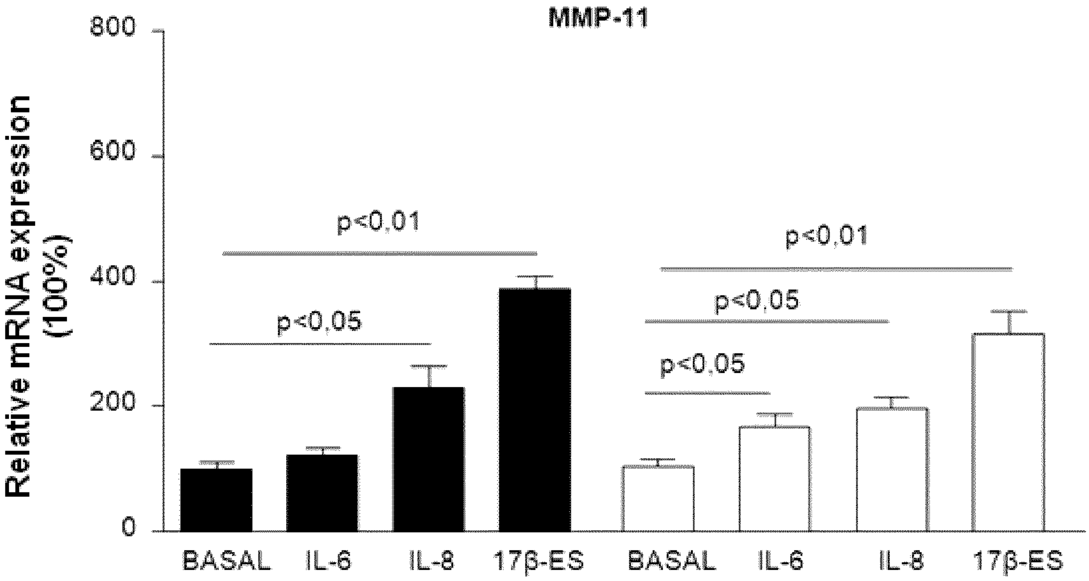


## Figure 15

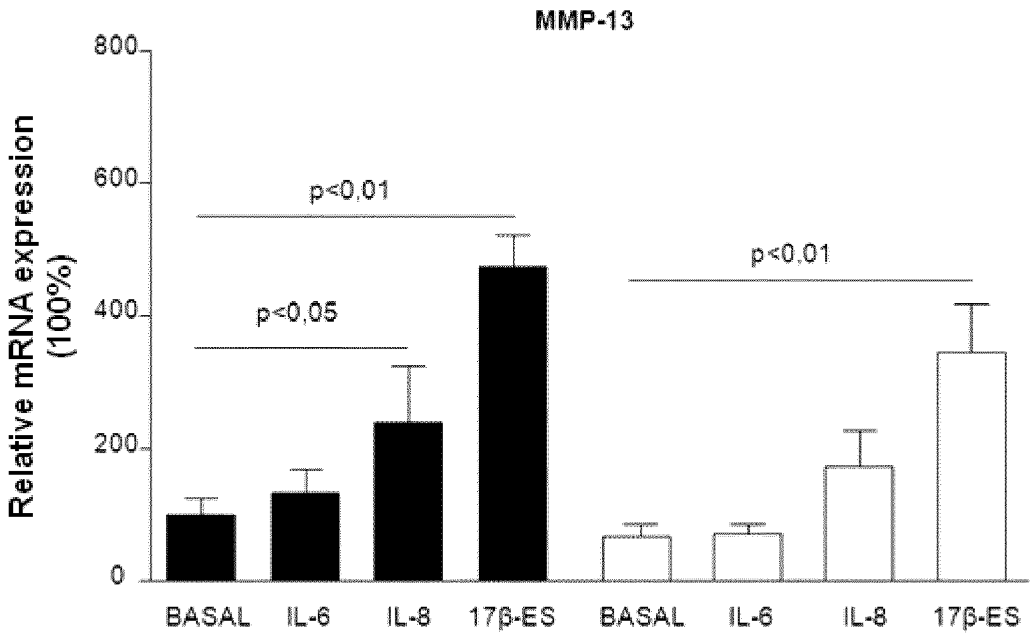

## Figure 16

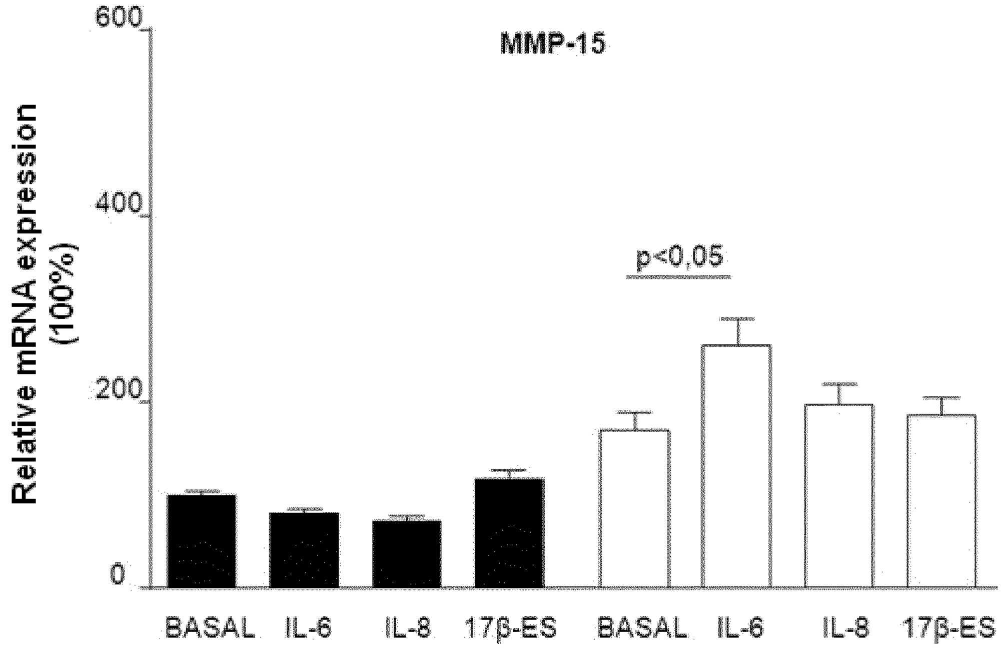


## Figure 17

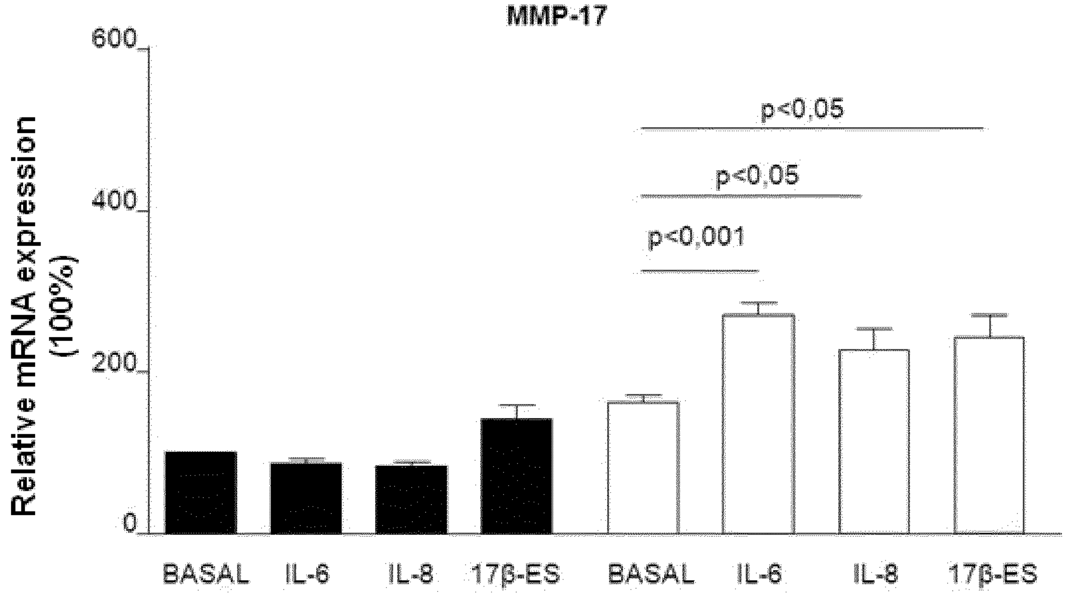

## Figure 18

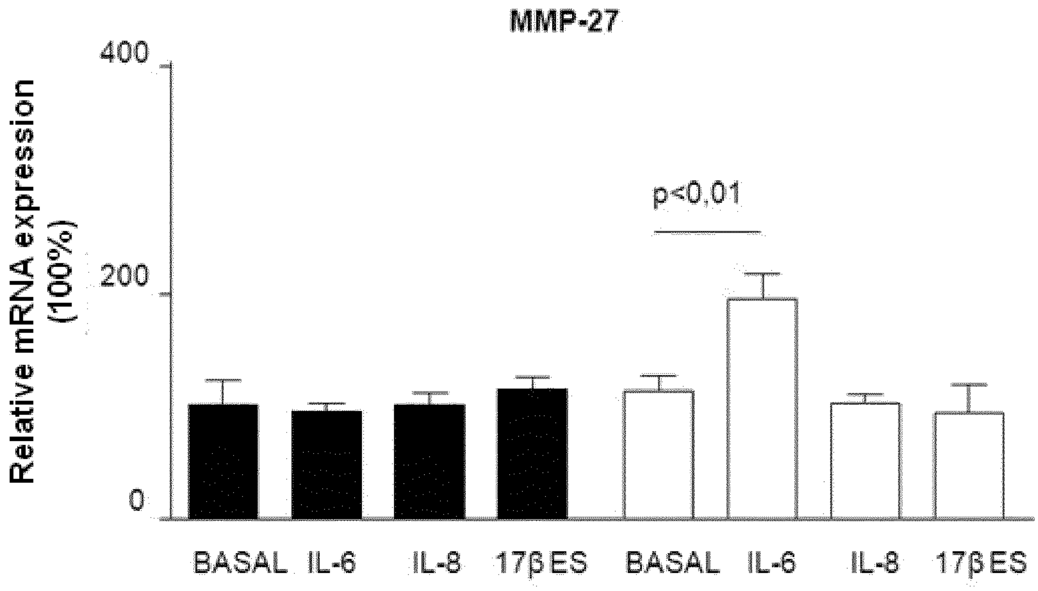


## Figure 19

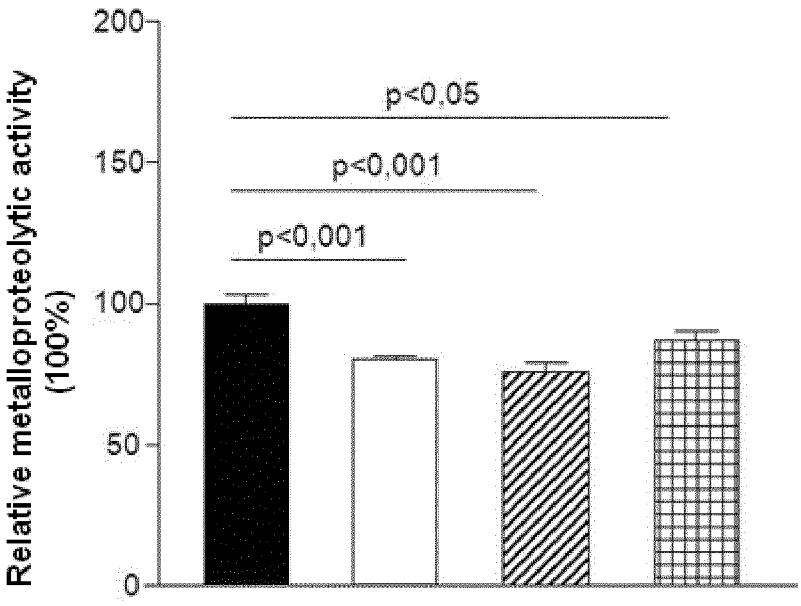

Figure 20

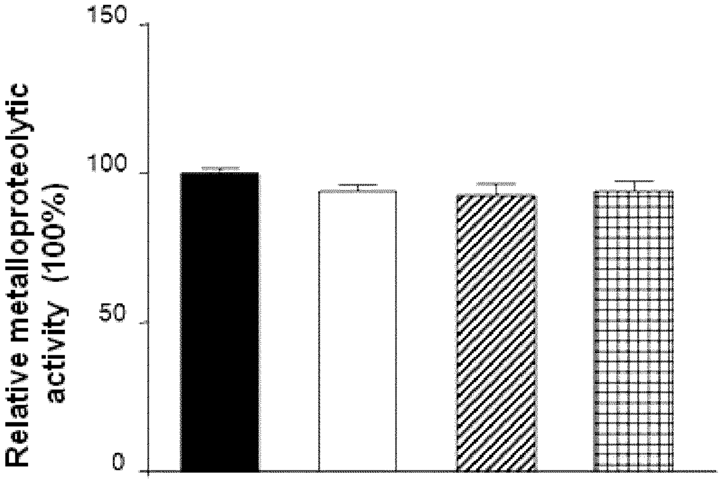


Figure 21

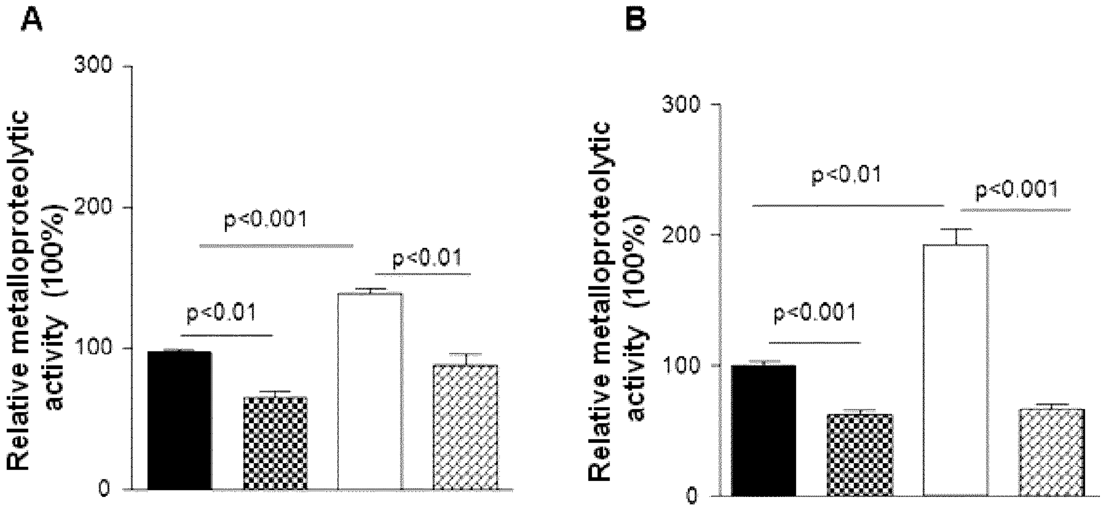

# Figure 22

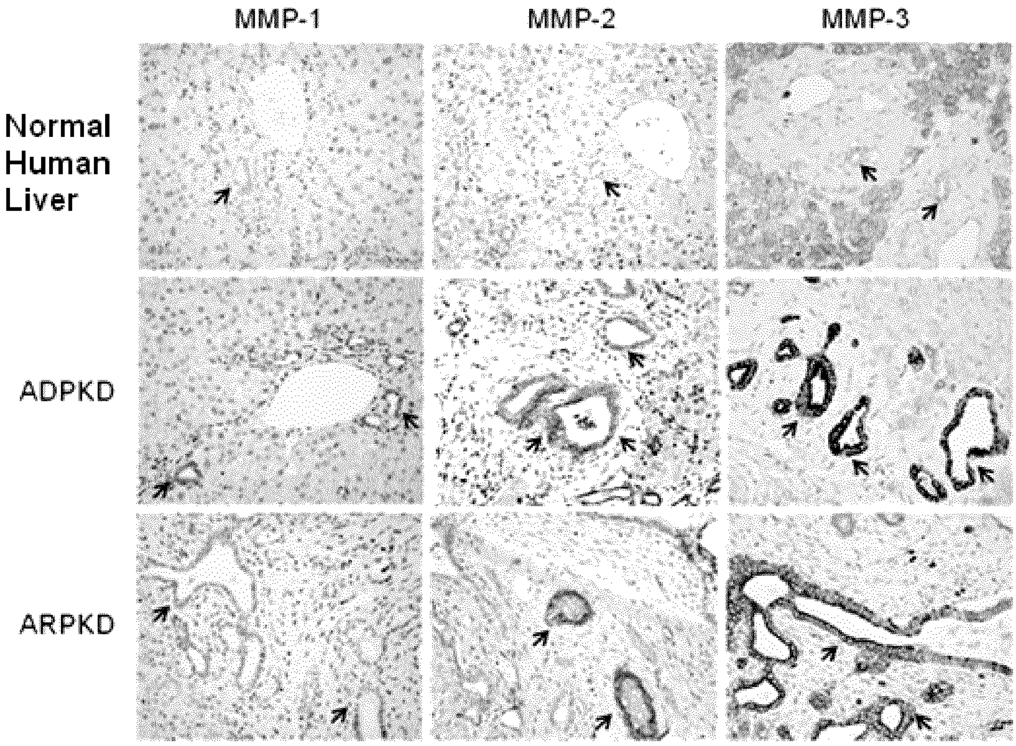


# Figure 23

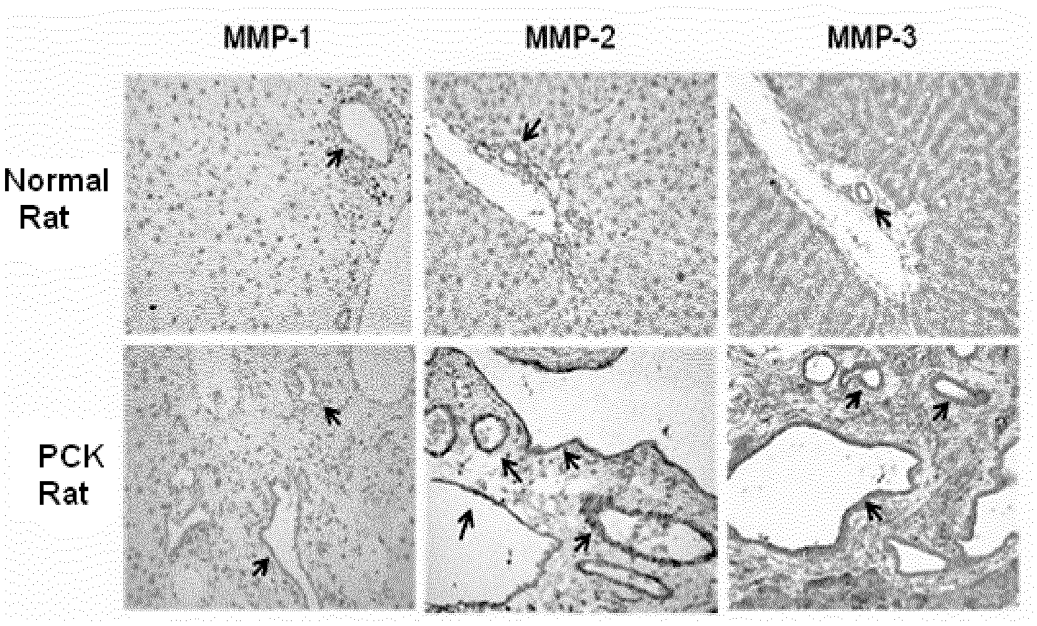

# Figure 24

A

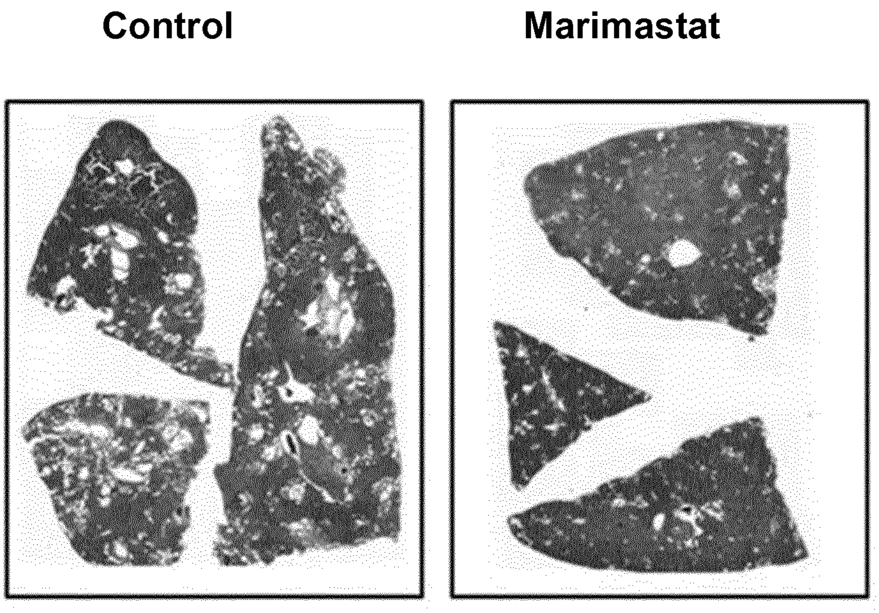
Control
Marimastat

B

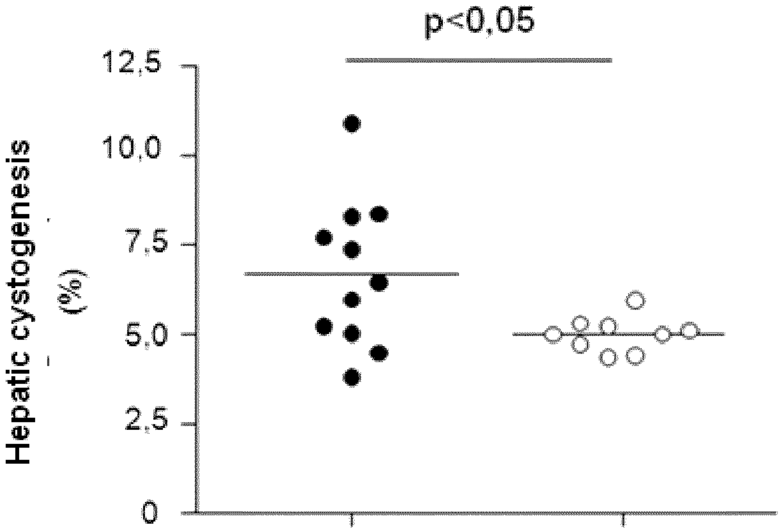
p<0,05
12,5
10,0
7,5
5,0
2,5
0
Hepatic cystogenesis
(%)

Figure 25

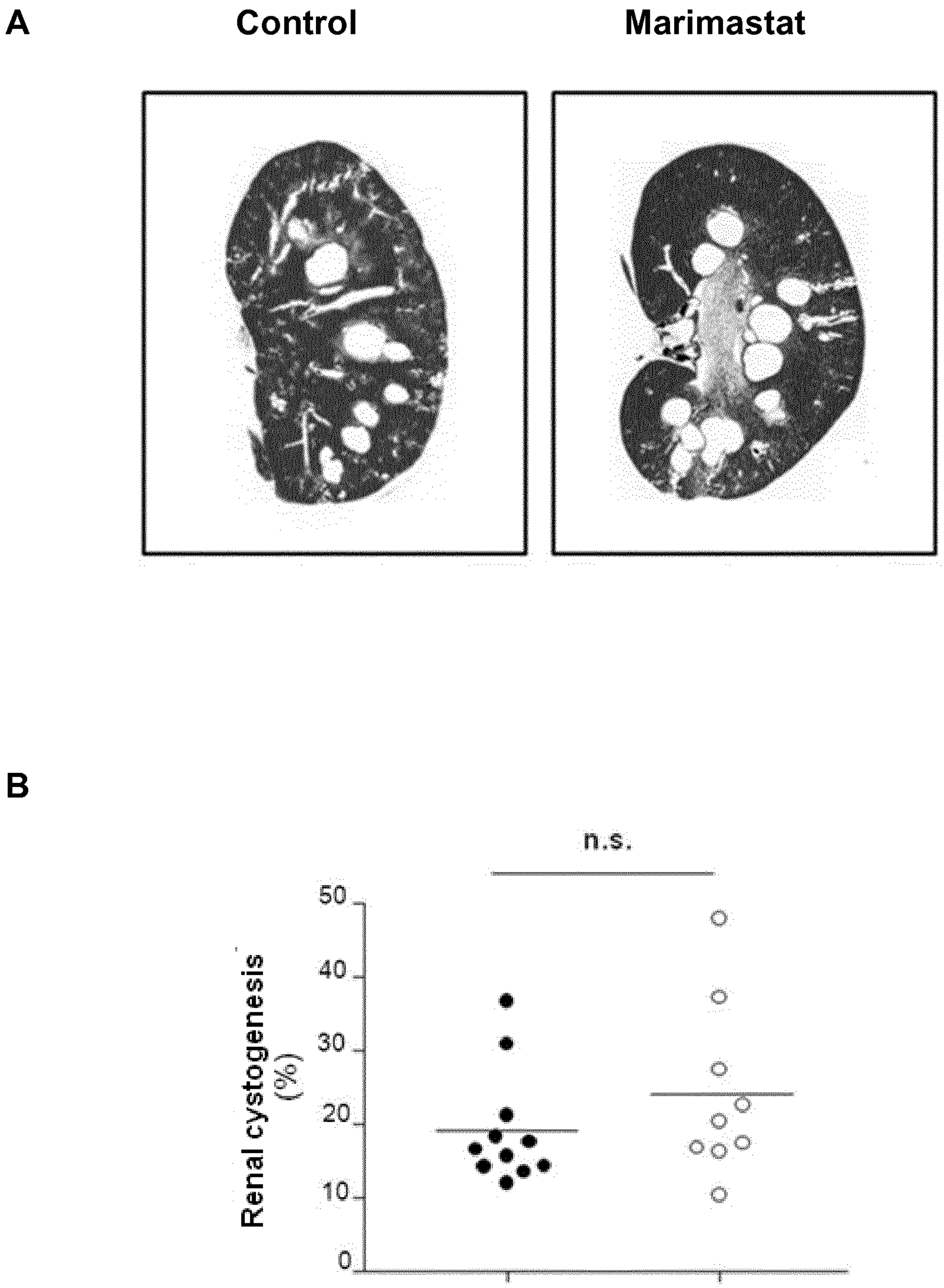

Figure 26

A Control Marimastat

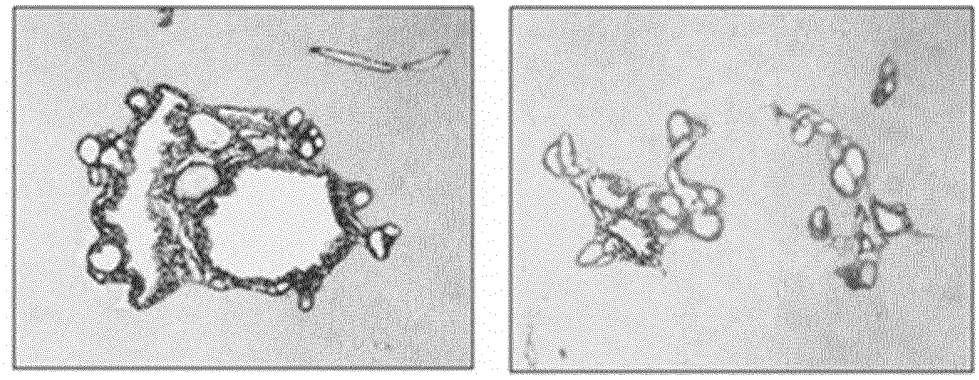

B Control Marimastat

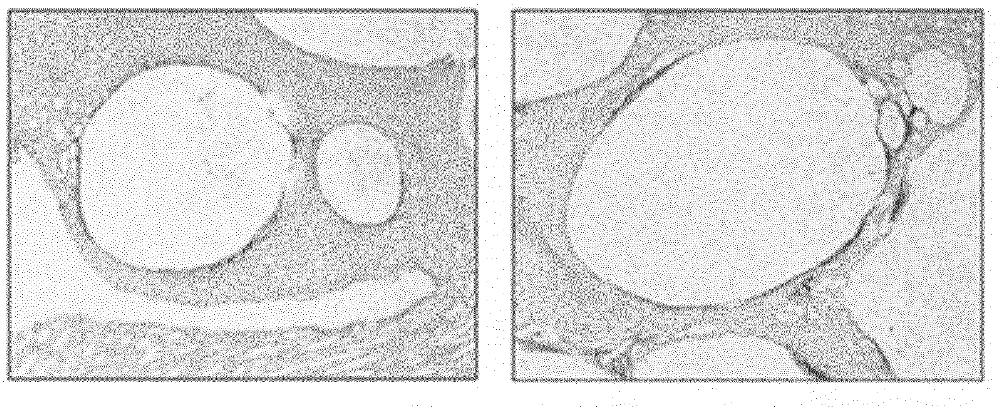

# Figure 27

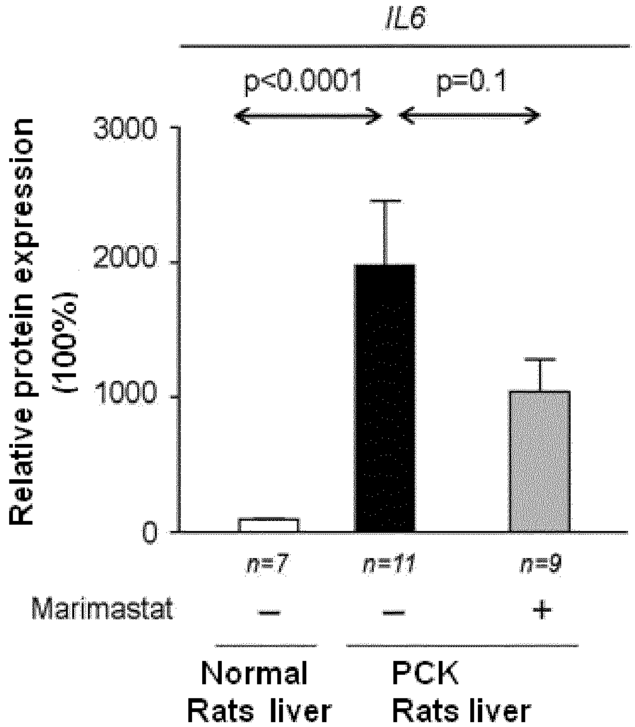
IL6
p<0.0001
p=0.1
Relative protein expression (100%)
3000
2000
1000
0
n=7
n=11
n=9
Marimastat
–
–
+
Normal Rats liver
PCK Rats liver

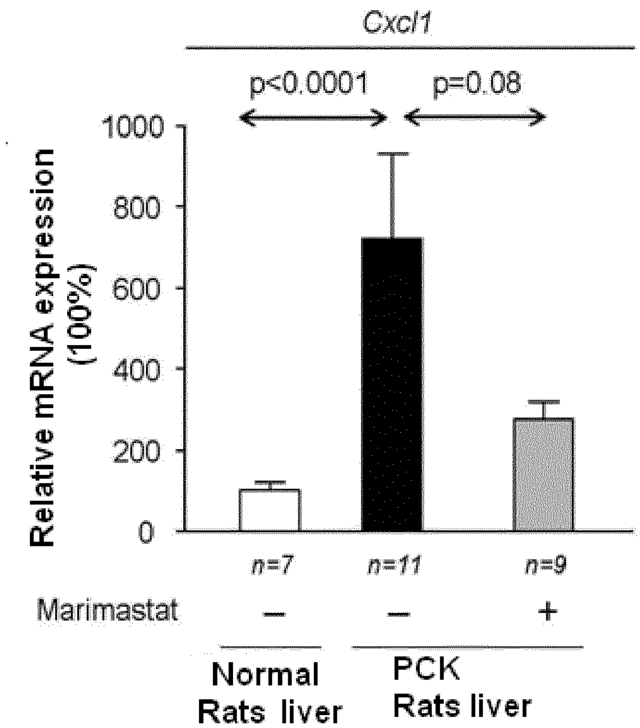
Cxcl1
p<0.0001
p=0.08
Relative mRNA expression (100%)
1000
800
600
400
200
0
n=7
n=11
n=9
Marimastat
–
–
+
Normal Rats liver
PCK Rats liver

## Figure 28

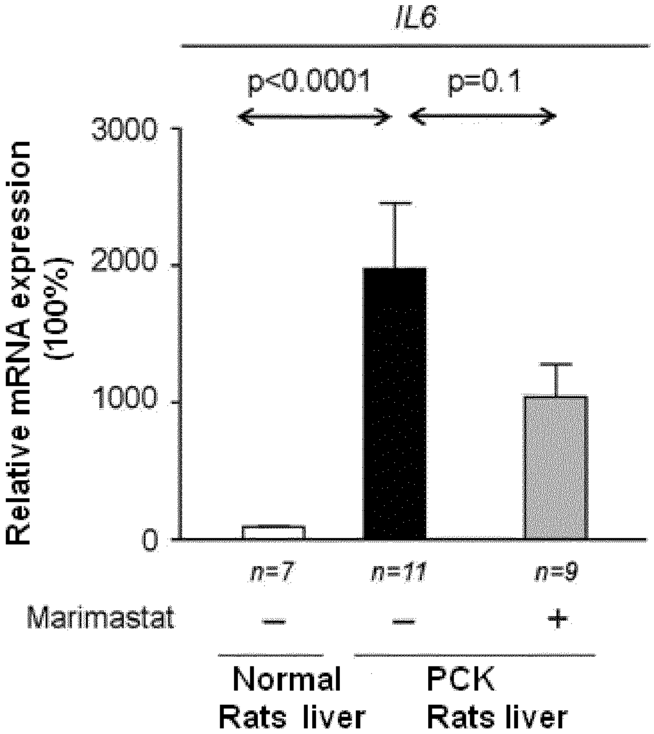
IL6
p<0.0001
p=0.1
Relative mRNA expression (100%)
3000
2000
1000
0
n=7
n=11
n=9
Marimastat
–
–
+
Normal Rats liver
PCK Rats liver

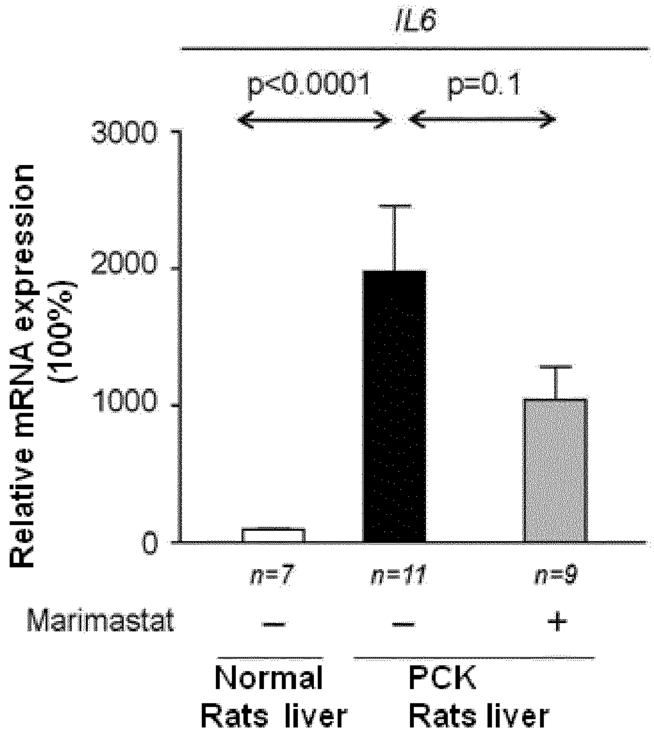
IL6
p<0.0001
p=0.1
Relative mRNA expression (100%)
3000
2000
1000
0
n=7
n=11
n=9
Marimastat
–
–
+
Normal Rats liver
PCK Rats liver

# Figure 29

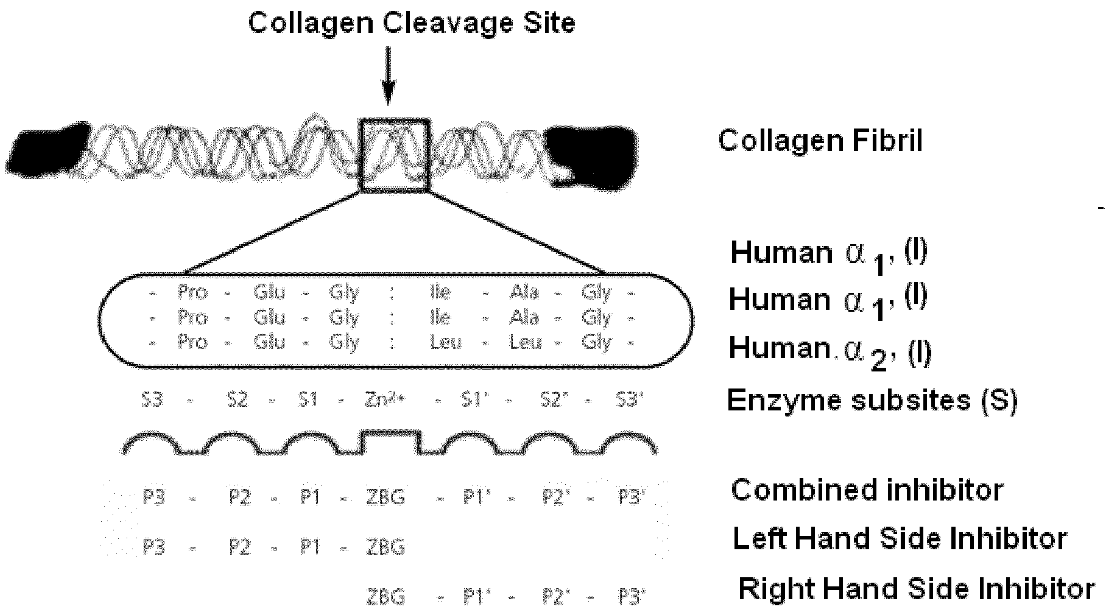
Collagen Cleavage Site
Collagen Fibril
- Pro - Glu - Gly : Ile - Ala - Gly -
- Pro - Glu - Gly : Ile - Ala - Gly -
- Pro - Glu - Gly : Leu - Leu - Gly -
Human α1, (I)
Human α1, (I)
Human α2, (I)
S3 - S2 - S1 - Zn2+ - S1' - S2' - S3'
Enzyme subsites (S)
P3 - P2 - P1 - ZBG - P1' - P2' - P3'
Combined inhibitor
P3 - P2 - P1 - ZBG
Left Hand Side Inhibitor
ZBG - P1' - P2' - P3'
Right Hand Side Inhibitor

## INTERNATIONAL SEARCH REPORT

International application No
PCT/ES2013/070824

**A. CLASSIFICATION OF SUBJECT MATTER**
INV. A61K31/16 A61K31/203 A61P1/16
ADD.

According to International Patent Classification (IPC) or to both national classification and IPC

**B. FIELDS SEARCHED**

Minimum documentation searched (classification system followed by classification symbols)
A61K A61P

Documentation searched other than minimum documentation to the extent that such documents are included in the fields searched

Electronic data base consulted during the international search (name of data base and, where practicable, search terms used)

EPO-Internal, WPI Data, BIOSIS, CHEM ABS Data, EMBASE

**C. DOCUMENTS CONSIDERED TO BE RELEVANT**

| Category* | Citation of document, with indication, where appropriate, of the relevant passages | Relevant to claim No. |
|---|---|---|
| X | UEMASU JIRO ET AL: "Regression of large hepatic cysts by minocycline hydrochloride in polycystic kidney disease", NEPHRON, vol. 69, no. 3, 1995, pages 365-366, XP055115178, ISSN: 0028-2766 the whole document abstract ----- | 1,2,6,8, 14 |
| X | LOPES HORACIO M N ET AL: "Treatment of benign hepatic cysts by instillation of tetracycline hydrochloride", HEPATO-GASTROENTEROLOGY, vol. 45, no. 20, March 1998 (1998-03), pages 496-499, XP009177722, ISSN: 0172-6390 abstract ----- | 1,2,6,8, 14 |
| | -/-- | |

[X] Further documents are listed in the continuation of Box C.

[X] See patent family annex.

* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 2 May 2014 | 09/05/2014 |

| Name and mailing address of the ISA/ | Authorized officer |
|---|---|
| European Patent Office, P.B. 5818 Patentlaan 2<br>NL - 2280 HV Rijswijk<br>Tel. (+31-70) 340-2040,<br>Fax: (+31-70) 340-3016 | Rodríguez-Palmero, M |

Form PCT/ISA/210 (second sheet) (April 2005)

## INTERNATIONAL SEARCH REPORT

International application No
PCT/ES2013/070824

C(Continuation). DOCUMENTS CONSIDERED TO BE RELEVANT

| Category* | Citation of document, with indication, where appropriate, of the relevant passages | Relevant to claim No. |
|---|---|---|
| X | EP 1 356 819 A1 (UNIV MEDISCH CENTRUM UTRECHT [NL]; UNIV UTRECHT [NL]) 29 October 2003 (2003-10-29) claims 9,13,14,17 ----- | 6-13 |
| X | HONGWEI HE ET AL: "Combination of retinoic acid and ursodeoxycholic acid attenuates liver injury in bile duct-ligated rats and human hepatic cells", HEPATOLOGY, vol. 53, no. 2, 10 February 2011 (2011-02-10), pages 548-557, XP055115201, ISSN: 0270-9139, DOI: 10.1002/hep.24047 abstract ----- | 6-9,12 |
| A | OBERMUELLER N ET AL: "A possible role for metalloproteinases in renal cyst development", AJP: RENAL PHYSIOLOGY, AMERICAN PHYSIOLOGICAL SOCIETY, vol. 280, March 2001 (2001-03), pages F540-F550, XP002223744, ISSN: 0363-6127 abstract page F543, column 1, paragraph 2 - column 2, paragraph 1 ----- | 1-14 |
| A | F. TEMMERMAN ET AL: "Systematic review: the pathophysiology and management of polycystic liver disease", ALIMENTARY PHARMACOLOGY & THERAPEUTICS, vol. 34, no. 7, 26 October 2011 (2011-10-26), pages 702-713, XP055115120, ISSN: 0269-2813, DOI: 10.1111/j.1365-2036.2011.04783.x tables 3,4 page 709, column 2, paragraph 2 - page 711, column 1, paragraph 1 ----- | 1-14 |
| T | MUNOZ-GARRIDO P ET AL: "Inhibition of metalloprotease hyperactivity in cystic cholangiocytes halts the development of polycystic liver diseases", GUT, vol. ONLINE FIRST, 16 January 2014 (2014-01-16), pages 1-10, XP009177685, ISSN: 0017-5749, DOI: 10.1136/GUTJNL-2013-305281 [retrieved on 2014-01-16] the whole document ----- | |

Form PCT/ISA/210 (continuation of second sheet) (April 2005)

## INTERNATIONAL SEARCH REPORT

Information on patent family members

International application No
PCT/ES2013/070824

| Patent document cited in search report | Publication date | Patent family member(s) | Publication date |
|---|---|---|---|
| EP 1356819 A1 | 29-10-2003 | NONE | |

Form PCT/ISA/210 (patent family annex) (April 2005)

## INTERNATIONAL SEARCH REPORT

International application No.
PCT/ES2013/070824

**Box No. I Nucleotide and/or amino acid sequence(s) (Continuation of item 1.c of the first sheet)**

1. With regard to any nucleotide and/or amino acid sequence disclosed in the international application and necessary to the claimed invention, the international search was carried out on the basis of:

   a. (means)
   - [ ] on paper
   - [X] in electronic form

   b. (time)
   - [X] in the international application as filed
   - [ ] together with the international application in electronic form
   - [ ] subsequently to this Authority for the purpose of search

2. [ ] In addition, in the case that more than one version or copy of a sequence listing and/or table relating thereto has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004110974 A
- WO 2004113279 A
- WO 2005026120 A
- US 6350885 B
- WO 9809940 A


### Non-patent literature cited in the description


- **MASYUK T. et al.** *Curr Opin Gastroenterol,* 2009, vol. 25, 265-271
- **BAÑALES JM et al.** *Pathophysiology of the intrahepatic biliary epithelium: Research SignPost,* 2008, 101-130
- **MASYUK T et al.** *Curr Opin Gastroenterol,* 2009, vol. 25, 265-271
- **TEMMERMAN F et al.** *Aliment Pharmacol Ther,* vol. 34, 702-713
- **BANALES JSM et al.** *The American Journal of Pathology,* 2008, vol. 173, 1637
- **BANALES JM et al.** *Hepatology,* 2009, vol. 49, 160-174
- **LEE S-O ; MASYUK T et al.** *The Journal of Clinical Investigation,* 2008, vol. 118, 3714
- **MASYUK TV et al.** *Gastroenterology,* 2007, vol. 132, 1104-1116
- **MASYUK TV et al.** *Molecular Biology of the Cell,* 2007
- **MASYUK TV et al.** *Curr Opin Gastroenterol,* 2009, vol. 25, 265-271
- **TEMMERMAN F et al.** *Aliment Pharmacol Ther.,* October 2011, vol. 34 (7), 702-13
- **VAN KEIMPEMA L et al.** *Gastroenterology,* 2009, vol. 137, 1661-8
- **HOGAN M et al.** *J Am Soc Nephrol,* 2010, vol. 21, 1052-61
- **QIAN Q et al.** *J Am Soc Nephrol,* 2008, vol. 19, 631-8
- **SERRA AL et al.** *N Engl J Med,* 2010, vol. 363, 820-9
- **WALZ G et al.** *N Engl J Med,* 2010, vol. 363, 830-40
- **WHITTAKER M. et al.** *Celltransmissions,* vol. 17 (1), 3-12
- **FISHER JF et al.** *Cancer Metastasis Rev.,* 2006, vol. 25, 115-136
- **ROTHENBERG ML et al.** *Oncologist,* 1998, vol. 3 (4), 271-274
- **SINGHMURA S.** *The Clinical Research Plus,* May 2011, 1
- **BANALES JM et al.** *Hepatology,* vol. 56, 687-697
- **AMURA CR et al.** *Am J Physiol Cell Physiol,* 2008, vol. 294, C786-796
- **NICHOLS MT et al.** *Hepatology,* 2004, vol. 40, 836
- **MASON SB et al.** *The Anatomical Record: Advances in Integrative Anatomy and Evolutionary Biology,* 2010, vol. 293, 1279-1288
- **NEMUNAITIS J et al.** *Clinical Cancer Research,* 1998, vol. 4, 1101-1109

SEQUENCE LISTING

<110> AdministraciÃ³n General de la Comunidad AutÃ³noma de Euskadi
Universidad de Salamanca

<120> USO DE INHIBIDORES DE METALOPROTEASAS EN EL TRATAMIENTO DE ENFERMEDADES HEPÃ\201TICAS POLIQUÃ\215STICAS

<130> PCT-06707

<150> ES201231850
<151> 2012-11-28

<160> 70

<170> BiSSAP 1.2

<210> 1
<211> 20
<212> DNA
<213> Artificial Sequence

<220>
<221> source
<222> 1..20
<223> /mol_type="unassigned DNA"
/note="Cebador sentido del gen AE2"
/organism="Artificial Sequence"

<400> 1
gctaagattt ggccatgagc 20

<210> 2
<211> 21
<212> DNA
<213> Artificial Sequence

<220>
<221> source
<222> 1..21
<223> /mol_type="unassigned DNA"
/note="Cebador antisentido del gen AE2"
/organism="Artificial Sequence"

<400> 2
cggtggtatt caaagtcttc c 21

<210> 3
<211> 20
<212> DNA
<213> Artificial Sequence

<220>
<221> source
<222> 1..20
<223> /mol_type="unassigned DNA"
/note="Cebador sentido del gen AQP1"
/organism="Artificial Sequence"

<400> 3
tatgcgtgct ggctactacc 20

<210> 4
<211> 20
<212> DNA
<213> Artificial Sequence

<220>
<221> source
<222> 1..20
<223> /mol_type="unassigned DNA"
/note="Cebador antisentido del gen AQP1"
/organism="Artificial Sequence"

<400> 4
agtcgtagat gagtacagcc 20

<210> 5
<211> 18
<212> DNA
<213> Artificial Sequence

<220>
<221> source
<222> 1..18
<223> /mol_type="unassigned DNA"
/note="Cebador sentido del gen CK19"
/organism="Artificial Sequence"

<400> 5
caacgaagct aaccatgc 18

<210> 6
<211> 20
<212> DNA
<213> Artificial Sequence

<220>
<221> source
<222> 1..20
<223> /mol_type="unassigned DNA"
/note="Cebador antisentido del gen CK19"
/organism="Artificial Sequence"

<400> 6
attggcttcg catgtcactc 20

<210> 7
<211> 20
<212> DNA
<213> Artificial Sequence

<220>
<221> source
<222> 1..20
<223> /mol_type="unassigned DNA"
/note="Cebador sentido del gen CK7"
/organism="Artificial Sequence"

<400> 7

atctttgagg cccagattgc 20

<210> 8
<211> 20
<212> DNA
<213> Artificial Sequence

<220>
<221> source
<222> 1..20
<223> /mol_type="unassigned DNA"
/note="Cebador antisentido del gen CK7"
/organism="Artificial Sequence"

<400> 8
ttgatctcat cattcagggc 20

<210> 9
<211> 20
<212> DNA
<213> Artificial Sequence

<220>
<221> source
<222> 1..20
<223> /mol_type="unassigned DNA"
/note="Cebador sentido del gen GADPH"
/organism="Artificial Sequence"

<400> 9
ccaaggtcat ccatgacaac 20

<210> 10
<211> 20
<212> DNA
<213> Artificial Sequence

<220>
<221> source
<222> 1..20
<223> /mol_type="unassigned DNA"
/note="Cebador antisentido del gen GADPH"
/organism="Artificial Sequence"

<400> 10
tgtcatacca ggaaatgagc 20

<210> 11
<211> 19
<212> DNA
<213> Artificial Sequence

<220>
<221> source
<222> 1..19
<223> /mol_type="unassigned DNA"
/note="Cebador sentido del gen MMP-1"
/organism="Artificial Sequence"

<400> 11
tcaaccaggc ccaggtatt 19

<210> 12
<211> 19
<212> DNA
<213> Artificial Sequence

<220>
<221> source
<222> 1..19
<223> /mol_type="unassigned DNA"
/note="Cebador antisentido del gen MMP-1"
/organism="Artificial Sequence"

<400> 12
tcaaccaggc ccaggtatt 19

<210> 13
<211> 24
<212> DNA
<213> Artificial Sequence

<220>
<221> source
<222> 1..24
<223> /mol_type="unassigned DNA"
/note="Cebador sentido del gen MMP-2"
/organism="Artificial Sequence"

<400> 13
tgggagaagg ccaagtggtc cgtg 24

<210> 14
<211> 24
<212> DNA
<213> Artificial Sequence

<220>
<221> source
<222> 1..24
<223> /mol_type="unassigned DNA"
/note="Cebador antisentido del gen MMP-2"
/organism="Artificial Sequence"

<400> 14
gtgcagctgt tgtactcctt gcca 24

<210> 15
<211> 20
<212> DNA
<213> Artificial Sequence

<220>
<221> source
<222> 1..20
<223> /mol_type="unassigned DNA"
/note="Cebador sentido del gen MMP-3"
/organism="Artificial Sequence"

<400> 15
ccagggatta atggagatgc 20

<210> 16
<211> 20
<212> DNA
<213> Artificial Sequence

<220>
<221> source
<222> 1..20
<223> /mol_type="unassigned DNA"
/note="Cebador antisentido del gen MMP-3"
/organism="Artificial Sequence"

<400> 16
atcttgagac aggcggaacc 20

<210> 17
<211> 19
<212> DNA
<213> Artificial Sequence

<220>
<221> source
<222> 1..19
<223> /mol_type="unassigned DNA"
/note="Cebador sentido del gen MMP-7"
/organism="Artificial Sequence"

<400> 17
tgtatgggga actgctgac 19

<210> 18
<211> 20
<212> DNA
<213> Artificial Sequence

<220>
<221> source
<222> 1..20
<223> /mol_type="unassigned DNA"
/note="Cebador antisentido del gen MMP-7"
/organism="Artificial Sequence"

<400> 18
tcatcctcat cgaagtgagc 20

<210> 19
<211> 20
<212> DNA
<213> Artificial Sequence

<220>
<221> source
<222> 1..20
<223> /mol_type="unassigned DNA"
/note="Cebador sentido del gen MMP-8"

/organism="Artificial Sequence"

<400> 19
ctcaagatga catcgatggc 20

<210> 20
<211> 20
<212> DNA
<213> Artificial Sequence

<220>
<221> source
<222> 1..20
<223> /mol_type="unassigned DNA"
/note="Cebador antisentido del gen MMP-8"
/organism="Artificial Sequence"

<400> 20
tctttgtagc tgaggatgcc 20

<210> 21
<211> 23
<212> DNA
<213> Artificial Sequence

<220>
<221> source
<222> 1..23
<223> /mol_type="unassigned DNA"
/note="Cebador sentido del gen MMP-9"
/organism="Artificial Sequence"

<400> 21
aaggatacag tttgttcctc gtg 23

<210> 22
<211> 22
<212> DNA
<213> Artificial Sequence

<220>
<221> source
<222> 1..22
<223> /mol_type="unassigned DNA"
/note="Cebador antisentido del gen MMP-9"
/organism="Artificial Sequence"

<400> 22
gcccctcagt gaagcggtac at 22

<210> 23
<211> 20
<212> DNA
<213> Artificial Sequence

<220>
<221> source
<222> 1..20
<223> /mol_type="unassigned DNA"

/note="Cebador sentido del gen MMP-10"
/organism="Artificial Sequence"

<400> 23
aagttaacag cagggacacc 20

<210> 24
<211> 21
<212> DNA
<213> Artificial Sequence

<220>
<221> source
<222> 1..21
<223> /mol_type="unassigned DNA"
/note="Cebador antisentido del gen MMP-10"
/organism="Artificial Sequence"

<400> 24
cttggataac ctgcttgtac c 21

<210> 25
<211> 20
<212> DNA
<213> Artificial Sequence

<220>
<221> source
<222> 1..20
<223> /mol_type="unassigned DNA"
/note="Cebador sentido del gen MMP-11"
/organism="Artificial Sequence"

<400> 25
agatctactt cttccgaggc 20

<210> 26
<211> 20
<212> DNA
<213> Artificial Sequence

<220>
<221> source
<222> 1..20
<223> /mol_type="unassigned DNA"
/note="Cebador antisentido del gen MMP-11"
/organism="Artificial Sequence"

<400> 26
tccagagcct tcaccttcac 20

<210> 27
<211> 20
<212> DNA
<213> Artificial Sequence

<220>
<221> source
<222> 1..20

```
<223> /mol_type="unassigned DNA"
      /note="Cebador sentido del gen MMP-13"
      /organism="Artificial Sequence"

<400> 27
tgacgatgta caagggatcc                                                  20


<210> 28
<211> 20
<212> DNA
<213> Artificial Sequence

<220>
<221> source
<222> 1..20
<223> /mol_type="unassigned DNA"
      /note="Cebador antisentido del gen MMP-13"
      /organism="Artificial Sequence"

<400> 28
cattgtttct cctcggagac                                                  20


<210> 29
<211> 20
<212> DNA
<213> Artificial Sequence

<220>
<221> source
<222> 1..20
<223> /mol_type="unassigned DNA"
      /note="Cebador sentido del gen MMP-14"
      /organism="Artificial Sequence"

<400> 29
gacacccact ttgactctgc                                                  20


<210> 30
<211> 20
<212> DNA
<213> Artificial Sequence

<220>
<221> source
<222> 1..20
<223> /mol_type="unassigned DNA"
      /note="Cebador antisentido del gen MMP-14"
      /organism="Artificial Sequence"

<400> 30
gcaaagtcga tgaggatgtc                                                  20


<210> 31
<211> 20
<212> DNA
<213> Artificial Sequence

<220>
<221> source
```

<222> 1..20
<223> /mol_type="unassigned DNA"
/note="Cebador sentido del gen MMP-15"
/organism="Artificial Sequence"

<400> 31
gaagtggaac aaccaccatc 20

<210> 32
<211> 20
<212> DNA
<213> Artificial Sequence

<220>
<221> source
<222> 1..20
<223> /mol_type="unassigned DNA"
/note="Cebador antisentido del gen MMP-15"
/organism="Artificial Sequence"

<400> 32
aagagtacca tgatgtcggc 20

<210> 33
<211> 20
<212> DNA
<213> Artificial Sequence

<220>
<221> source
<222> 1..20
<223> /mol_type="unassigned DNA"
/note="Cebador sentido del gen MMP-16"
/organism="Artificial Sequence"

<400> 33
agcagtccat gaactgggac 20

<210> 34
<211> 20
<212> DNA
<213> Artificial Sequence

<220>
<221> source
<222> 1..20
<223> /mol_type="unassigned DNA"
/note="Cebador antisentido del gen MMP-16"
/organism="Artificial Sequence"

<400> 34
aggaatcttg tcaggtggac 20

<210> 35
<211> 20
<212> DNA
<213> Artificial Sequence

<220>

<221> source
<222> 1..20
<223> /mol_type="unassigned DNA"
/note="Cebador sentido del gen MMP-17"
/organism="Artificial Sequence"

<400> 35
caagtggaac aagaggaacc 20

<210> 36
<211> 20
<212> DNA
<213> Artificial Sequence

<220>
<221> source
<222> 1..20
<223> /mol_type="unassigned DNA"
/note="Cebador antisentido del gen MMP-17"
/organism="Artificial Sequence"

<400> 36
gagaagtcga tctggatgtc 20

<210> 37
<211> 20
<212> DNA
<213> Artificial Sequence

<220>
<221> source
<222> 1..20
<223> /mol_type="unassigned DNA"
/note="Cebador sentido del gen MMP-18"
/organism="Artificial Sequence"

<400> 37
gcaagagaca tgatcatgcc 20

<210> 38
<211> 20
<212> DNA
<213> Artificial Sequence

<220>
<221> source
<222> 1..20
<223> /mol_type="unassigned DNA"
/note="Cebador antisentido del gen MMP-18"
/organism="Artificial Sequence"

<400> 38
ttgtgccctg actcatgagc 20

<210> 39
<211> 20
<212> DNA
<213> Artificial Sequence

<220>
<221> source
<222> 1..20
<223> /mol_type="unassigned DNA"
/note="Cebador sentido del gen MMP-24"
/organism="Artificial Sequence"

<400> 39
catgcagatg catgctctgc 20

<210> 40
<211> 19
<212> DNA
<213> Artificial Sequence

<220>
<221> source
<222> 1..19
<223> /mol_type="unassigned DNA"
/note="Cebador antisentido del gen MMP-24"
/organism="Artificial Sequence"

<400> 40
ccagaactgc tcgatctgc 19

<210> 41
<211> 20
<212> DNA
<213> Artificial Sequence

<220>
<221> source
<222> 1..20
<223> /mol_type="unassigned DNA"
/note="Cebador sentido del gen MMP-25"
/organism="Artificial Sequence"

<400> 41
aaagtcatgc agaggttcgc 20

<210> 42
<211> 20
<212> DNA
<213> Artificial Sequence

<220>
<221> source
<222> 1..20
<223> /mol_type="unassigned DNA"
/note="Cebador antisentido del gen MMP-25"
/organism="Artificial Sequence"

<400> 42
gaagacaaac atctcccctc 20

<210> 43
<211> 20
<212> DNA
<213> Artificial Sequence

```
<220>
<221> source
<222> 1..20
<223> /mol_type="unassigned DNA"
      /note="Cebador sentido del gen MMP-26"
      /organism="Artificial Sequence"

<400> 43
ctaccagtac atggagacgc                                                   20


<210> 44
<211> 20
<212> DNA
<213> Artificial Sequence

<220>
<221> source
<222> 1..20
<223> /mol_type="unassigned DNA"
      /note="Cebador antisentido del gen MMP-26"
      /organism="Artificial Sequence"

<400> 44
gcatggccta agataccacc                                                   20


<210> 45
<211> 20
<212> DNA
<213> Artificial Sequence

<220>
<221> source
<222> 1..20
<223> /mol_type="unassigned DNA"
      /note="Cebador sentido del gen MMP-27"
      /organism="Artificial Sequence"

<400> 45
atcattggag tgagagagcc                                                   20


<210> 46
<211> 20
<212> DNA
<213> Artificial Sequence

<220>
<221> source
<222> 1..20
<223> /mol_type="unassigned DNA"
      /note="Cebador antisentido del gen MMP-27"
      /organism="Artificial Sequence"

<400> 46
atcattggag tgagagagcc                                                   20


<210> 47
<211> 20
<212> DNA
```

<213> Artificial Sequence

<220>
<221> source
<222> 1..20
<223> /mol_type="unassigned DNA"
/note="Cebador sentido del gen TIMP-1"
/organism="Artificial Sequence"

<400> 47
gaaactagaa gccaacagcc 20

<210> 48
<211> 20
<212> DNA
<213> Artificial Sequence

<220>
<221> source
<222> 1..20
<223> /mol_type="unassigned DNA"
/note="Cebador antisentido del gen TIMP-1"
/organism="Artificial Sequence"

<400> 48
agcaatgagt gccactctgc 20

<210> 49
<211> 20
<212> DNA
<213> Artificial Sequence

<220>
<221> source
<222> 1..20
<223> /mol_type="unassigned DNA"
/note="Cebador sentido del gen TIMP-2"
/organism="Artificial Sequence"

<400> 49
caaccctatc aagaggatcc 20

<210> 50
<211> 20
<212> DNA
<213> Artificial Sequence

<220>
<221> source
<222> 1..20
<223> /mol_type="unassigned DNA"
/note="Cebador antisentido del gen TIMP-2"
/organism="Artificial Sequence"

<400> 50
agtcacagag ggtgatgtgc 20

<210> 51
<211> 20

```
<212> DNA
<213> Artificial Sequence

<220>
<221> source
<222> 1..20
<223> /mol_type="unassigned DNA"
      /note="Cebador sentido del gen TIMP-3"
      /organism="Artificial Sequence"

<400> 51
caacaagtac cagtacctgc                                                   20


<210> 52
<211> 20
<212> DNA
<213> Artificial Sequence

<220>
<221> source
<222> 1..20
<223> /mol_type="unassigned DNA"
      /note="Cebador antisentido del gen TIMP-3"
      /organism="Artificial Sequence"

<400> 52
tggaagtcac aaagcaaggc                                                   20


<210> 53
<211> 20
<212> DNA
<213> Artificial Sequence

<220>
<221> source
<222> 1..20
<223> /mol_type="unassigned DNA"
      /note="Cebador sentido del gen TIMP-4"
      /organism="Artificial Sequence"

<400> 53
tgcggatact tccacaggtc                                                   20


<210> 54
<211> 20
<212> DNA
<213> Artificial Sequence

<220>
<221> source
<222> 1..20
<223> /mol_type="unassigned DNA"
      /note="Cebador antisentido del gen TIMP-4"
      /organism="Artificial Sequence"

<400> 54
ggtacagggt actgtgtagc                                                   20


<210> 55
```

<211> 20
<212> DNA
<213> Artificial Sequence

<220>
<221> source
<222> 1..20
<223> /mol_type="unassigned DNA"
/note="Cebador sentido del gen Col1a1"
/organism="Artificial Sequence"

<400> 55
gactgtccca acccccaaaa 20

<210> 56
<211> 20
<212> DNA
<213> Artificial Sequence

<220>
<221> source
<222> 1..20
<223> /mol_type="unassigned DNA"
/note="Cebador antisentido del gen Col1a1"
/organism="Artificial Sequence"

<400> 56
cttgggtccc tcgactccta 20

<210> 57
<211> 22
<212> DNA
<213> Artificial Sequence

<220>
<221> source
<222> 1..22
<223> /mol_type="unassigned DNA"
/note="Cebador sentido del gen alpha-Sma"
/organism="Artificial Sequence"

<400> 57
cgccatcagg aacctcgaga ag 22

<210> 58
<211> 20
<212> DNA
<213> Artificial Sequence

<220>
<221> source
<222> 1..20
<223> /mol_type="unassigned DNA"
/note="Cebador antisentido del gen alpha-Sma"
/organism="Artificial Sequence"

<400> 58
atcatcacca gcaaagcccg 20

<210> 59
<211> 20
<212> DNA
<213> Artificial Sequence

<220>
<221> source
<222> 1..20
<223> /mol_type="unassigned DNA"
/note="Cebador sentido del gen TgfÎ²1"
/organism="Artificial Sequence"

<400> 59
ctgctgaccc ccactgatac 20

<210> 60
<211> 20
<212> DNA
<213> Artificial Sequence

<220>
<221> source
<222> 1..20
<223> /mol_type="unassigned DNA"
/note="Cebador antisentido del gen TgfÎ²1"
/organism="Artificial Sequence"

<400> 60
agccctgtat tccgtctcct 20

<210> 61
<211> 20
<212> DNA
<213> Artificial Sequence

<220>
<221> source
<222> 1..20
<223> /mol_type="unassigned DNA"
/note="Cebador sentido del gen Ctgf"
/organism="Artificial Sequence"

<400> 61
ctagctgcct accgactgga 20

<210> 62
<211> 20
<212> DNA
<213> Artificial Sequence

<220>
<221> source
<222> 1..20
<223> /mol_type="unassigned DNA"
/note="Cebador antisentido del gen Ctgf"
/organism="Artificial Sequence"

<400> 62
gcccatccca caggtcttag 20

<210> 63
<211> 20
<212> DNA
<213> Artificial Sequence

<220>
<221> source
<222> 1..20
<223> /mol_type="unassigned DNA"
/note="Cebador sentido del gen Cxcl1"
/organism="Artificial Sequence"

<400> 63
actcaagaat ggtcgcgagg 20

<210> 64
<211> 20
<212> DNA
<213> Artificial Sequence

<220>
<221> source
<222> 1..20
<223> /mol_type="unassigned DNA"
/note="Cebador antisentido del gen Cxcl1"
/organism="Artificial Sequence"

<400> 64
acgccatcgg tgcaatctat 20

<210> 65
<211> 20
<212> DNA
<213> Artificial Sequence

<220>
<221> source
<222> 1..20
<223> /mol_type="unassigned DNA"
/note="Cebador sentido del gen IL1Î²"
/organism="Artificial Sequence"

<400> 65
aggctgacag accccaaaag 20

<210> 66
<211> 20
<212> DNA
<213> Artificial Sequence

<220>
<221> source
<222> 1..20
<223> /mol_type="unassigned DNA"
/note="Cebador antisentido del gen IL1Î²"
/organism="Artificial Sequence"

<400> 66
ctccacgggc aagacatagg 20

<210> 67
<211> 20
<212> DNA
<213> Artificial Sequence

<220>
<221> source
<222> 1..20
<223> /mol_type="unassigned DNA"
/note="Cebador sentido del gen Gapdh"
/organism="Artificial Sequence"

<400> 67
tgtgaacgga tttggccgta 20

<210> 68
<211> 20
<212> DNA
<213> Artificial Sequence

<220>
<221> source
<222> 1..20
<223> /mol_type="unassigned DNA"
/note="Cebador antisentido del gen Gapdh"
/organism="Artificial Sequence"

<400> 68
atgaaggggt cgttgatggc 20

<210> 69
<211> 20
<212> DNA
<213> Artificial Sequence

<220>
<221> source
<222> 1..20
<223> /mol_type="unassigned DNA"
/note="Cebador sentido del gen IL-6"
/organism="Artificial Sequence"

<400> 69
cattctgtct cgagcccacc 20

<210> 70
<211> 20
<212> DNA
<213> Artificial Sequence

<220>
<221> source
<222> 1..20
<223> /mol_type="unassigned DNA"
/note="Cebador antisentido del gen IL-6"
/organism="Artificial Sequence"

<400> 70

```
agtcccaaga aggcaactgg                                                   20
```